# EUROPÄISCHE PATENTSCHRIFT

(11) **EP 3 548 895 B1**
(45) Veröffentlichungstag und Bekanntmachung des Hinweises auf die Patenterteilung: **03.07.2024**
(21) Anmeldenummer: 16822599.3
(22) Anmeldetag: 30.11.2016
(51) Int. Cl.: G01N 33/68

(54) **AUFBEREITUNG LEBENDIGER, MIKROBIELLER PROBEN UND MIKROORGANISMEN FÜR ANSCHLIESSENDE MASSENSPEKTROMETRISCHE MESSUNG UND AUSWERTUNG**
PREPARING LIVE MICROBIAL SAMPLES AND MICROORGANISMS FOR SUBSEQUENT MASS-SPECROMETRIC MEASUREMENT AND EVALUATION
PREPARATION D'ECHANTILLONS MICROBIENS VIVANTS ET DE MICROORGANISMES POUR EFFECTUER ENSUITE UNE MESURE ET UNE EVALUATION PAR SPECTROMETRIE DE MASSE

(43) Veröffentlichungstag der Anmeldung: 09.10.2019
(73) Patentinhaber: Bruker Daltonics GmbH & Co. KG, 28359 Bremen (DE)
(72) Erfinder: IDELEVICH, Evgeny, 48149 Münster (DE); BECKER, Karsten, 48366 Laer (DE)
(74) Vertreter: Boßmeyer, Jens
(86) Internationale Anmeldenummer: PCT/DE2016/100561
(87) Internationale Veröffentlichungsnummer: WO 2018/099500

(56) Entgegenhaltungen:
- WO-A1-2012/023845
- WO-A1-2016/016580
- US-A1- 2015 086 971
- E.A. IDELEVICH ET AL: "Direct blood culturing on solid medium outperforms an automated continuously monitored broth-based blood culture system in terms of time to identification and susceptibility testing", NEW MICROBES AND NEW INFECTIONS, Bd. 10, 1. März 2016 (2016-03-01), Seiten 19-24, XP055374332, ISSN: 2052-2975, DOI: 10.1016/j.nmni.2015.12.004
- J. WATROUS ET AL: "Mass spectral molecular networking of living microbial colonies", PROCEEDINGS NATIONAL ACADEMY OF SCIENCES PNAS, Bd. 109, Nr. 26, 14. Mai 2012 (2012-05-14) , Seiten E1743-E1752, XP055377081, US ISSN: 0027-8424, DOI: 10.1073/pnas.1203689109
- Franco Basile: "Rapid Sample Preparation for Microorganism Analysis by Mass Spectrometry" In: "ACS Symposium Series", 1. Januar 2011 (2011-01-01), American Chemical Society/Oxford University Press, US, XP055377083, ISSN: 0097-6156 Bd. 1065, Seiten 5-34, DOI: 10.1021/bk-2011-1065.ch002, (S 7, Abs 1 ff)(S 16, Abs 2); Zusammenfassung
- Bruker: "MALDI Biotyper Consumables Added Convenience Enhances Assay Productivity", , 1. Januar 2015 (2015-01-01), XP055377091, Gefunden im Internet: URL:https://www.bruker.com/fileadmin/user_ upload/8-PDF-Docs/Separations_MassSpectrom etry/Literature/Brochures/1838970_MBT_Cons umables_RUO_brochure_08-2015_eBook.pdf [gefunden am 2017-05-30]
- Bruker: "Instructions for Use MSP 96 polished steel BC targets Reusable MALDI targets for MALDI-TOF-MS analysis", Revision C, 1. Dezember 2015 (2015-12-01), XP055377088, Gefunden im Internet: URL:https://www.bruker.com/fileadmin/user_ upload/8-PDF-Docs/Separations_MassSpectrom etry/Literature/Brochures/1838970_MBT_Cons umables_RUO_brochure_08-2015_eBook.pdf [gefunden am 2017-05-30]

## Beschreibung

### Gebiet der Erfindung

Die Erfindung betrifft Verfahren zur Aufbereitung lebendiger, mikrobieller Proben und Mikroorganismen für eine anschließende massenspektrometrische Messung und Auswertung. Erkenntnisse, die sich aus einer solchen Messung ableiten lassen, können insbesondere der beschleunigten Identifizierung von Mikroorganismen in der mikrobiellen Probe nach Art/Unterart und/oder der schnellen Bestimmung von Resistenz/Empfindlichkeit der Mikroorganismen gegenüber antimikrobiellen Substanzen und/oder der weiteren Charakterisierung von Mikroorganismen dienen; zum Beispiel hinsichtlich Pathogenität, Virulenz und Metabolismus. Gemäß einer bevorzugten Ausführungsform der Erfindung findet die Aufbereitung insbesondere direkt auf einem massenspektrometrischen Probenträger statt.

### Hintergrund der Erfindung

Infektionskrankheiten stellen nach wie vor ein Hauptproblem in der Medizin dar. Infektionen können eigenständig vorkommen, entwickeln sich aber insbesondere als Komplikationen anderer Erkrankungen oder als Folge von immunsupprimierenden Therapien und/oder des Einsatzes von Fremdkörpermaterialien am Patienten. In den letzten Jahren waren unter anderem der Fortschritt der modernen Medizin und der damit verbundene Anstieg von komplizierten Eingriffen und immunsupprimierenden Behandlungen sowie die Verwendung von Fremdkörpern für eine Erhöhung der Infektionsraten verantwortlich. Beispielsweise können in diesem Zusammenhang Transplantationen von soliden Organen und Knochenmarkttransplantationen genannt werden.

Besorgniserregend ist insbesondere der Anstieg von multiresistenten Erregern; zum Beispiel bei Bakterien (u.a. MRSA - Methicillin-resistenter *Staphylococcus aureus;* VRE - Vancomycin-resistente Enterokokken; Ciprofloxacin-, Meropenem- oder Tobramycin-resistente *Pseudomonas aeruginosa*) oder Pilzen (u.a. Fluconazol- oder Voriconazol-resistente *Candida albicans*)*.* Die durch solche Erreger verursachten Infektionen sind besonders schwer antibiotisch zu behandeln. Da die initial verabreichten antimikrobiellen Mittel im Rahmen einer so genannten "empirischen" oder "kalkulierten" Therapie multiresistente Erreger in ihrem Aktivitätsspektrum in der Regel nicht erfassen, ist es für den Erfolg der Behandlung entscheidend, die Resistenzen frühzeitig zu detektieren. Eine schnelle Erkennung von resistenten Mikroorganismen ermöglicht eine rechtzeitige Umstellung auf gegen diese Erreger wirksame antimikrobielle Substanzen. Diese werden im Folgenden vereinfacht als Antibiotika bezeichnet; es sind aber nicht nur gegen Bakterien wirksame Substanzen sondern auch Mittel gegen Pilze und andere Mikroorganismen gemeint. Eine derartige frühzeitige Umstellung auf eine korrekte antimikrobielle Therapie kann für den Erfolg der Behandlung entscheidend sein.

Derzeit fehlen insbesondere phänotypische (gemeint sind kulturbasierte) Testsysteme oder Einzeltests, die ein Ergebnis der Empfindlichkeitstestung innerhalb weniger Stunden liefern können. Phänotypische Resistenz bedeutet, dass der Mikroorganismus trotz Gegenwart eines Antibiotikums wächst. Bei der phänotypischen Antibiotikaempfindlichkeit wird das Wachstum in Anwesenheit des getesteten Antibiotikums gehemmt, sofern dieses in ausreichender Konzentration appliziert wurde. Die phänotypische Empfindlichkeitstestung stellt den Goldstandard dar; unter anderem, da die Testergebnisse unabhängig von den zu Grunde liegenden Resistenzmechanismen generiert werden. Molekularbiologisch können zwar innerhalb kurzer Zeit bestimmte Resistenzgene nachgewiesen werden; zum Beispiel durch Polymerase-Kettenreaktion (*polymerase chain reaction* - PCR). Dieser Nachweis ist allerdings nur für eine Auswahl der Resistenzmechanismen möglich; die anderen Resistenzmechanismen werden nicht erfasst. Außerdem erfassen derartige molekulare Tests nur bereits bekannte genetisch kodierte Resistenzmechanismen. Es ist also keine zuverlässige Aussage bezüglich der Empfindlichkeit eines Erregers gegenüber einem Antibiotikum möglich, wenn ein bestimmtes Resistenzgen nicht detektiert wird. Außerdem gelingt mit diesen Methoden auch bei weitem nicht immer eine zuverlässige Vorhersage der phänotypischen Resistenz, falls ein Resistenzgen nachgewiesen wird. Denn die Ausprägung der Genexpression kann unterschiedlich sein; und der Mikroorganismus kann trotz Vorhandensein des Gens phänotypisch empfindlich auf das Antibiotikum reagieren.

Ferner ist für eine Vielzahl der Resistenzmechanismen eine Gen-Detektion nicht möglich. Zu den Methoden, die in der Lage sind, bestimmte Resistenzmechanismen phänotypisch schnell zu detektieren, gehört zum Beispiel der Nachweis von den durch einige Bakterien produzierten β-Laktamasen. β-Laktamasen sind bakterielle Enzyme, die β-Laktam-Antibiotika spalten und dadurch unwirksam machen können. Der Nachweis kann durch die Detektion der β-Laktam-Spaltung erfolgen, beispielsweise durch Farbänderung eines pH-Indikators oder mittels MALDI-TOF MS (matrix-unterstützte Laserdesorption/Ionisation - MALDI; TOF - *time-of-flight,* Flugzeit; Massenspektrometrie - MS). Bei MALDI-TOF MS bestimmt man massenspektrometrisch das ungespaltene β-Laktam und/oder dessen Spaltprodukte. Diese Methoden können zwar in bestimmten Situationen vorteilhaft sein, haben aber auch den generellen Nachteil, dass nur ein bestimmter Resistenzmechanismus nachgewiesen wird und eine allgemeine, endgültige Aussage über die Empfindlichkeit oder Resistenz eines Erregers nicht möglich ist.

Es besteht somit ein akuter Bedarf an Methoden, die einerseits eine wachstumsbasierte, phänotypische Empfindlichkeitstestung und damit eine generelle Aussage wie bei üblichen Testmethoden ermöglichen, andererseits aber deutlich schneller sind als die üblichen Verfahren. Es wäre das generelle Ziel für solche Schnelltests, das Ergebnis innerhalb von wenigen Stunden zu liefern, d.h. zum Beispiel innerhalb von 1-4 Stunden. Die Erreichbarkeit dieser zeitlichen Ziele ist zum einen von der Testmethode abhängig, und zum anderen von den Eigenschaften der zu testenden Mikroorganismen; beispielsweise der Wachstumsgeschwindigkeit.

Die vor kurzem entwickelte MALDI-TOF MS-basierte Methode "MBT ASTRA" zur Empfindlichkeitstestung durch Quantifizierung des mikrobiellen Wachstums unter Verwendung eines internen Standards demonstrierte die Realisierbarkeit einer generellen wachstumsbasierten Empfindlichkeitstestung mittels MALDI-TOF MS (Lange et al., Journal of Clinical Microbiology, December 2014, Volume 52, Number 12, p. 4155-4162; und K. Sparbier et al. / Methods 104 (2016) 48-54). Allerdings benötigt das Verfahren in der bisher beschriebenen Form mehrere Arbeitsschritte, die einen deutlichen Arbeitsaufwand im Labor bedeuten. Dieser Aufwand kann die Akzeptanz der Methode mindern und dadurch die Einführung dieser für Patienten grundsätzlich vorteilhaften Methode in der Routine-Diagnostik behindern, gegebenenfalls sogar verhindern.

Die Studie "Direct blood culturing on solid medium outperforms an automated continuously monitored broth-based blood culture system in terms of time to identification and susceptibility testing" von E. A. Idelevich et al. (New Microbe and New Infect 2016; 10: 19-24) erläutert eine Untersuchung, ob eine Lyse-Zentrifugations-Blutkultur-Methode, kombiniert mit einer matrix-unterstützten Laser-Desorptions-Ionisations-Flugzeit-Massenspektrometrie (MALDI-TOF MS)-Identifikation und Vitek 2 AST, einen Zeitvorteil im Vergleich zu einem automatisierten, auf Nährbouillon basierenden Blutkultur-System bietet.

In der Arbeit "Mass spectral molecular networking of living microbial colonies" von Jeramie Watrous et al. (PNAS | Published online www.pnas.org/cgi/doi/10.1073/pnas.1203689109, May 14, 2012 | E1743-E1752) wird eine Integration von zwei Methoden vorgestellt: Nanospray-Desorption-Elektrospray-Ionisation (nano-DESI) Massenspektrometrie zur direkten chemischen Überwachung lebender mikrobieller Populationen in Verbindung mit der Erzeugung molekularer Netzwerke.

Das Buchkapitel 2 "Rapid Sample Preparation for Microorganism Analysis by Mass Spectrometry" von Franco Basile (in: ACS Symposium Series, 2011, American Chemical Society/Oxford University Press, US, Bd. 1065, Seiten 5-34) gibt einen Überblick über verschiedene Probenvorbereitungsschritte, die im Zusammenhang mit der Schnellanalyse von Mikroorganismen mit verschiedenen Massenspektrometrie-Techniken verwendet werden.

Die Produktinformationsbroschüre "MALDI Biotyper Consumables - Added Convenience Enhances Assay Productivity", August 2015 (www.bruker.com/fileadmin/userupload/8-PDF-Docs/Separations MassSpectrometry/LiteratureBrochures/1838970 MBT Consumables RUO brochure 08-2015 eBook.pdf) beschreibt verschiedene Produktangebote des Herstellers.

Die Gebrauchsanleitung "Instructions for Use - MSP 96 polished steel BC targets - Reusable MALDI targets for MALDI-TOF-MS analysis", Revision C, Dezember 2015 (www.bruker.com/fileadmin/userupload/8-PDF-Docs/Separations MassSpectrometry/Literature/Brochures/1838970 MBT Consumables RUO brochure 08-2015 eBook.pdf") widmet sich der Handhabung von MALDI-Probenträgern.

Die Patentveröffentlichung US 2015/0086971 A1 offenbart Verfahren und Vorrichtungen zur Lokalisierung und Auswahl einer Kolonie von Mikroorganismen auf einer Kulturschale und zur Identifizierung von Mikroorganismen in der ausgewählten Kolonie unter Verwendung von MALDI. Umfasst sind die automatisierten Schritte des Auffindens und Auswählens einer Kolonie von Mikroorganismen auf einer Kulturschale; des Gewinnens einer Probe der ausgewählten Kolonie von Mikroorganismen; des Auftragens von mindestens einem Teil der Probe der ausgewählten Kolonie von Mikroorganismen auf eine Zielplatte; und des Überführens der Zielplatte mit der Probe in eine Vorrichtung zur Durchführung von MALDI zur Identifizierung der Probe der ausgewählten Kolonie von Mikroorganismen. Eine Probe einer Kolonie von Mikroorganismen wird automatisch auf einem Ablagerungsfleck abgelagert, so dass die Probe höchstens etwa die Hälfte des einen Ablagerungsflecks der Zielplatte bedeckt. Eine Suspension einer Probe von Mikroorganismen wird automatisch hergestellt, indem die Probe mit einem Entnahmewerkzeug automatisch entnommen und das Entnahmewerkzeug mit der Probe in eine Suspension eingetaucht wird, woraufhin das Entnahmewerkzeug nur in vertikaler Richtung vibriert wird, um die Probe aus dem Entnahmewerkzeug zu lösen.

Die Patentveröffentlichung WO 2016/016580 A1 betrifft ein Verfahren zur Vorbereitung einer Fläche auf einer Analyseplatte, um mindestens eine Charakterisierung einer Population von mindestens einem Mikroorganismus in Gegenwart mindestens eines antimikrobiellen Mittels mittels Massenspektrometrie nach der als MALDI bekannten Technik der matrixunterstützten Laserdesorptions/Ionisations-Massenspektrometrie durchzuführen. Umfasst sind die folgenden aufeinanderfolgenden Schritte: - einen Schritt, der darin besteht, eine Analyseplatte zu verwenden, die für die Charakterisierung mittels der MALDI-Technik bestimmt ist und mindestens einen Analysebereich umfasst, der ein antimikrobielles Mittel enthält; - einen Schritt, der darin besteht, die Population mindestens eines Mikroorganismus auf dem Analysebereich in Kontakt mit dem antimikrobiellen Mittel abzulagern; - einen Inkubationsschritt, der darin besteht, die Analyseplatte während einer ausreichenden Zeitspanne und unter Bedingungen aufzubewahren, die ausreichen, um die Wechselwirkung des antimikrobiellen Mittels mit dem vorhandenen Mikroorganismus zu ermöglichen; und - einen Schritt, der darin besteht, auf dem Analysebereich eine für die MALDI-Technik geeignete Matrix abzulagern.

Die Patentveröffentlichung WO 2012/023845 A1 betrifft ein Verfahren zur Charakterisierung der Antibiotikaresistenz eines Mikroorganismus, wobei das Verfahren die folgenden Schritte umfasst: (a) Bereitstellen eines Referenzmassenspektrums einer antimikrobiellen Verbindung, ihres enzymatischen Modifikationsprodukts, ihres molekularen Ziels oder einer Substratverbindung ihres modifizierenden Enzyms; (b) Aussetzen eines Mikroorganismus, eines Zelllysats davon oder eines Wachstumsmedium-Überstands davon der antimikrobiellen Verbindung oder der Substratverbindung in wässriger Flüssigkeit, um dadurch eine exponierte Probe bereitzustellen; (c) Erfassen eines Massenspektrums der exponierten Probe; (d) Vergleichen des in Schritt (c) erfassten Massenspektrums mit dem Referenzmassenspektrum aus Schritt (a), und (e) Bestimmen aus dem Vergleich, ob eine Modifikation der antimikrobiellen Verbindung, ihres Modifikationsprodukts oder ihres molekularen Ziels oder des Substrats nach der Exposition stattgefunden hat, und Feststellen, dass der Mikroorganismus potenziell resistent gegen die antimikrobielle Verbindung ist, wenn die Modifikation beobachtet wird.

Angesichts der vorstehenden Ausführungen besteht ein Bedarf, Verfahren bereitzustellen, mit denen die Aufbereitung lebendiger, mikrobieller Proben für die anschließende massenspektrometrische Messung vereinfacht und beschleunigt werden kann. Weitere von der Erfindung zu lösende Aufgaben ergeben sich für den Fachmann ohne weiteres bei der Lektüre der nachfolgenden Offenbarung.

### Zusammenfassung der Erfindung

Die hier beschriebenen Verfahren stellen eine alternative Methode für eine sehr schnelle und einfache MS-basierte mikrobielle Messung dar; beispielsweise bezüglich der Identifizierung von Art/Unterart und/oder einer Resistenz/Empfindlichkeitstestung und/oder weiterer Erregercharakterisierung. Die Offenbarung bezieht sich insbesondere auf das Verfahren der Probenverarbeitung/Probenaufbereitung sowie auf Algorithmen der Datenauswertung.

Gemäß einem ersten bevorzugten Aspekt betrifft die vorliegende Offenbarung ein Verfahren zur Aufbereitung lebendiger, mikrobieller Proben für eine anschließende massenspektrometrische Messung, das die folgenden Schritte aufweist: (a) Bereitstellen eines flachen Probenträgers, der mehrere Probenflecken umfasst (sogenannte "Spots"); (b) Aufbringen wenigstens einer lebendigen, mikrobiellen Probe in einem Nährmedium-Tropfen auf wenigstens einen der Probenflecken; (c) Verbringen des Probenträgers in einen Bebrütungsraum mit definierter Atmosphäre für eine vorbestimmte Zeitspanne, um Mikroorganismenwachstum anzuregen; (d) Entfernen von Restflüssigkeit des Nährmedium-Tropfens nach der vorbestimmten Zeitspanne, um eine Mikroorganismenablagerung auf dem Probenfleck freizulegen; (e) Präparieren des Probenflecks für eine desorbierende Ionisierung; (f) Überführen des Probenträgers in eine Desorptions-Ionenquelle eines Massenspektrometers, Erzeugen von Ionen aus dem präparierten Probenfleck und Aufnehmen wenigstens eines zugehörigen Massenspektrums; und (g) Abgleichen des aufgenommenen Massenspektrums mit einem Referenzdatensatz, um wenigstens eine Eigenschaft der mikrobiellen Probe zu ermitteln.

Der erste bevorzugte Aspekt der Offenbarung beruht insbesondere auf der neuen und überraschenden Erkenntnis, dass ein flacher massenspektrometrischer Probenträger, der in einer geeigneten Ionenquelle als Substrat für die Ionisierung der aufbereiteten Proben dient, in einem vorgelagerten Schritt bereits als Substrat für eine wachstumsfördernde Bebrütung von Mikroorganismen fungieren kann. Diese Doppelfunktion erleichtert den Arbeitsablauf im Labor enorm und verkürzt die erforderliche Zeit für den diagnostischen Arbeitsprozess, weil sich umständliche und fehleranfällige, händische Probenübertragungsschritte vermeiden lassen und separate Bearbeitungsgefäße wie Mikrotitrationsplatten überflüssig werden. Diese prozessuale Erleichterung kann dazu beitragen, die schnelle, verlässliche und ausführlich validierte massenspektrometrische Vermessung von Mikroorganismen in der klinischen Diagnostik zu etablieren.

In verschiedenen Ausführungsformen kann der Referenzdatensatz Referenzspektren aufweisen, die einer Bibliothek früher aufgenommener Massenspektren entnommen werden, wobei die wenigstens eine Eigenschaft aus Schritt (g) im Rahmen einer Identifizierung Art oder Unterart von Mikroorganismen in der mikrobiellen Probe umfassen kann. In dieser einfachen Variante fungiert der Nährmedium-Tropfen als reiner Wachstumsreaktor auf dem massenspektrometrischen Probenträger. Fachleute werden anerkennen, dass sich Mikroorganismen in einer Flüssigkeit unter anderem wegen der grundsätzlich allseitigen Umspülung mit Nährmedium schneller vermehren können als auf einem flächigen Nährmedium wie einer Agarschicht in einer Petri-Schale, so dass das vorgeschlagene Verfahren einen Zeitvorteil bringt, der sich im klinischen Umfeld als für den Patienten überlebenswichtig erweisen kann.

In bestimmten Fällen kann der Referenzdatensatz aus dem in Schritt (f) aufgenommenen Massenspektrum von darin enthaltenen Massensignalen, die nicht ursprünglich von Mikroorganismen stammen, abgeleitet werden. Als Beispiel seien Massensignale einer (oder mehrerer) im Zuge der Aufbereitung der mikrobiellen Probe hinzugefügten Referenzsubstanz(en) genannt (interner Standard), die beispielsweise der Quantifizierung dienen können.

In bevorzugten Ausführungsformen wird die gleiche mikrobielle Probe in Schritt (b) parallel auf mehrere Probenflecken aufgebracht, wobei die Nährmedium-Tropfen mal eine antimikrobielle Substanz enthalten und mal nicht. Ein massenspektrometrischer Probenträger eignet sich besonders gut für eine umfangreiche Resistenz/Empfindlichkeitstestung, weil er Platz genug bietet, um das Wachstum von Mikroorganismen in Gegenwart unterschiedlicher antimikrobieller Substanzen (oder gegenüber der gleichen antimikrobiellen Substanz bei unterschiedlichen Konzentrationen) gleichzeitig zu überwachen. Die Frage, ob ein Mikroorganismus auf eine bestimmte antimikrobielle Substanz empfindlich reagiert (bzw. ab welcher Konzentration er es tut), was beispielsweise deren Wirksamkeit als Medikament indizieren würde, kann also sehr schnell und verlässlich geklärt werden.

In besonderen Ausführungen des Verfahrens können mehrere Nährmedium-Tropfen mit antimikrobieller Substanz mal einen Enzym-Hemmer enthalten und mal nicht. Von großem klinisch-therapeutischen Interesse kann es sein, wenn ein β-Laktamase-Hemmer als Enzym-Hemmer verwendet wird. Diese Erweiterung der Resistenz/Empfindlichkeitstestung erlaubt eine Aussage über eine β-Laktamase-begründete Resistenz, wenn der untersuchte Mikroorganismus in Gegenwart des β-Laktam-Antibiotikums im Wachstum nicht gehemmt wird, in Gegenwart einer Kombination aus β-Laktam-Antibiotikum und β-Laktamase-Hemmer jedoch schon.

In verschiedenen Ausführungsformen kann der Referenzdatensatz ein zeitnah aufgenommenes Massenspektrum eines Probenflecks sein, auf den in Schritt (b) ein Nährmedium-Tropfen ohne antimikrobielle Substanz oder Enzym-Hemmer aufgebracht worden ist; und die wenigstens eine Eigenschaft aus Schritt (g) kann im Rahmen einer Charakterisierung eine Resistenz/Empfindlichkeit von Mikroorganismen in der mikrobiellen Probe gegenüber der antimikrobiellen Substanz bzw. einer Kombination aus antimikrobieller Substanz und Enzym-Hemmer umfassen. Auf diese Weise lässt sich insbesondere die minimale Hemmkonzentration eines Antibiotikums bezüglich der Mikroorganismen ermitteln, indem in Schritt (b) mehrere Nährmedium-Tropfen mit jeweils verschiedenen Konzentrationen der antimikrobiellen Substanz (und/oder ggf. des Hemmers) aufgebracht werden und der Grad der Wirksamkeit entlang einer Reihe ansteigender oder abnehmender Konzentrationen bewertet wird.

In einer weiteren Ausführungsform kann der Referenzdatensatz ein Massenspektrum eines Probenflecks auf einem zweiten Probenträger sein, wobei die Mikroorganismen für das als Referenzdatensatz verwendete Massenspektrum kürzer bebrütet wurden, ggf. auch völlig ohne Bebrütung. Die mikrobielle Probe wird dabei bevorzugt in einem Nährmedium-Tropfen ohne antimikrobielle Substanz oder ohne eine Kombination aus antimikrobieller Substanz und Enzym-Hemmer auf den Probenfleck aufgebracht. Die anfangs auf den Probenflecken aufgebrachten Mengen an mikrobiellen Zellen für die Massenspektren der mikrobiellen Probe und des Referenzdatensatzes sind vorzugsweise gleich.

Vorzugsweise wird die wenigstens eine Eigenschaft in Schritt (g) aus einem Unterschied im Mikroorganismenwachstum abgeleitet, das sich in der Ausprägung oder Intensität der Mikroorganismen-spezifischen Massensignal-Signatur im aufgenommenen Massenspektrum wiederfindet; je nachdem, ob eine Wachstumshemmung durch eine antimikrobielle Substanz allein oder in Verbindung mit einem Enzym-Hemmer gefunden werden konnte oder nicht. In einer einfachen Variante lässt sich das Mikroorganismenwachstum an Hand einer gelungenen Identifizierung der Art bewerten, sofern die vor der Bebrütung abgemessene Menge der Mikroorganismen in der mikrobiellen Probe nicht für eine verlässliche Identifizierung ausreicht. Als Beispiel für ein wohlbekanntes Identifizierungsverfahren sei der MALDI Biotyper^{®}-Algorithmus genannt, der eine vertretbar verlässliche Identifizierung einer Art eines Mikroorganismus bestätigt, sofern die berechnete Ähnlichkeitskennzahl (sogenannter "log(Score)") größer oder gleich 1,7 ist. Ein hoher Grad von Verlässlichkeit wird mit Ähnlichkeitskennzahlen größer oder gleich 2,0 erzielt.

In verschiedenen Ausführungsformen kann die mikrobielle Probe in Schritt (b) in dem Nährmedium-Tropfen derart bemessen werden, dass eine Menge leicht unterhalb der Nachweisgrenze der massenspektrometrischen Messung liegt. Auf diese Weise lässt sich insbesondere die Zeitspanne, die der Probenträger in dem Bebrütungsraum verbringen muss, um Mikroorganismenwachstum anzuregen, auf ein Mindestmaß verringern. Denn schon Wachstum, das die Nachweisgrenze erreicht oder leicht übersteigt, kann im Vergleich zu einer Messung, die außer Hintergrundsignalen keine aussagekräftigen Daten enthält, als Indiz für die Gegenwart oder eine Eigenschaftsausprägung des Mikroorganismus gedeutet werden.

In verschiedenen Ausführungsformen lassen sich Temperatur und Feuchtigkeit in dem Bebrütungsraum in Schritt (c) auf etwa 36°C (für eine Bebrütung oder Empfindlichkeitstestung ggf. notwendig oder sogar vorgeschrieben) bzw. nahe einer Sättigung einstellen, um bestmögliche oder geforderte Wachstumsbedingungen für die zu untersuchenden Mikroorganismen zu schaffen. Das Ziel sollte allgemein sein, in dem Bebrütungsraum solche Bedingungen zu schaffen, die Wachstumsunterschiede am deutlichsten zutage treten lassen. Die Temperatur von 36°C entspricht etwa der menschlichen Körpertemperatur und ist für diejenigen Mikroorganismen geeignet, die sich auf den Menschen als Wirt spezialisiert haben. In der Veterinär-, Lebensmittel- oder Umweltdiagnostik zum Beispiel können aber durchaus auch andere Temperaturen als am besten geeignet erkannt werden; je nachdem, welches Wirts- oder Umgebungsmilieu von dem Mikroorganismus bevorzugt wird. Die hohe Luftfeuchtigkeit nahe 100% dient insbesondere dazu, ein verfrühtes Verdunsten des Nährmedium-Tropfens zu verhindern, damit das für das Mikroorganismenwachstum zu Verfügung stehende Flüssigkeitsvolumen über die vorbestimmte Zeitspanne von üblicherweise einigen Stunden, in der sich der Probenträger im Bebrütungsraum befindet (generell 1-18 Stunden), annähernd erhalten bleibt.

In verschiedenen Ausführungsformen kann das Entfernen von Restflüssigkeit (nach dem Bebrütungsschritt) ein Abtupfen eines Tropfenüberstandes beispielsweise mittels eines saugfähigen Materials oder ein Abpipettieren umfassen. Diese Varianten haben den Vorteil, dass Reste der in dem Nährmedium-Tropfen befindlichen Substanzen zusammen mit der Flüssigkeit weitgehend von dem Probenfleck entfernt werden, was den chemischen Untergrund in der anschließenden massenspektrometrischen Messung verringern kann. Ebenso ist es jedoch möglich, die Flüssigkeit in kurzer Zeit verdampfen zu lassen, beispielsweise unter Zuhilfenahme eines Heißluftgebläses. In einem solchen Fall scheiden sich die Substanzen in dem flüssigen Nährmedium auf der Mikroorganismenablagerung ab und werden anschließend wenigstens teilweise zusammen mit dieser für die folgende massenspektrometrische Messung präpariert.

In verschiedenen Ausführungsformen kann das Präparieren in Schritt (e) eine vorbereitende Extraktion mikrobieller Proteine/Peptide aus der Mikroorganismenablagerung und/oder ein Waschen der Mikroorganismenablagerung und/oder eine Einbettung der Mikroorganismenablagerung in eine Laserlicht-absorbierende Matrixsubstanz zwecks anschließender Ionisierung per matrix-unterstützter Laserdesorption (MALDI) umfassen. Im Extraktionsfall kann die Anzahl Mikroorganismen-spezifischer Massensignale im aufgenommen Massenspektrum erhöht und damit die Aussagekraft der Messung verbessert werden; insbesondere wenn eine Identifizierung nach Art/Unterart des untersuchten Mikroorganismus angestrebt wird. Ein oder mehrere Waschschritte eignen sich insbesondere zum Entfernen von nahezu allgegenwärtigen Salzen aus der Mikroorganismenablagerung, die ansonsten die Ionisierungseffizienz verschlechtern können. Das Aufbereitungsverfahren lässt sich auf diese Weise weiter optimieren. Beispiele für Matrixsubstanzen sind 2,5-Dihydroxybenzoesäure, Sinapinsäure oder α-Cyano-4-hydroxy-Zimtsäure. Das MALDI-Verfahren hat sich als sehr wichtiges und verlässliches Werkzeug bei der Ionenbasierten Untersuchung von Mikroorganismen erwiesen. Gleichzeitig erlaubt es eine gepulste Ionenerzeugung, die sich gut für eine Flugzeit-dispersive Aufnahme von Massenspektren eignet.

Es sind allerdings auch andere Desorptions-Ionisierungsarten zur Verwendung mit dem beschriebenen Verfahren vorstellbar, die kein Auftragen einer Matrixsubstanz erfordern; beispielsweise Desorptions-Elektrosprüh-Ionisierung (DESI) oder Ionisierung mittels Sekundärionen-Massenspektrometrie (SIMS). In ganz speziellen Fällen kann die Präparation in Schritt (e) auch nur eine kurze Wartezeit von zum Beispiel wenigen Minuten ohne weitere Behandlung der Mikroorganismenablagerung umfassen.

Vorteilhafterweise werden die Massenspektren in Schritt (f) Flugzeit-dispersiv aufgenommen. Flugzeit-Massenspektrometer können gegenwärtig wegen ihres hohen Auflösungsvermögens, der schnellen Messzeit und ihrer breiten Massenakzeptanz sowohl in der klinischen als auch außer-klinischen Mikroorganismenuntersuchung als "Goldstandard" angesehen werden. Als Beispiele für Massenspektrometer, die nach dem Flugzeitprinzip arbeiten, seien diejenigen der kommerziell erhältlichen Serie microflex^{®} von Bruker Daltonik GmbH genannt.

In verschiedenen Ausführungsformen kann in Schritt (b) die mikrobielle Probe (i) als Suspension in dem Nährmedium-Tropfen auf dem wenigstens einen Probenfleck dispensiert oder (ii) zuerst in Zellform auf dem wenigstens einen Probenflecken abgelegt und anschließend in einen dispensierten Nährmedium-Tropfen eingetaucht werden. In weiteren Varianten können auch die antimikrobiellen Substanzen (ggf. auch die Enzym-Hemmer) entweder zusammen mit der mikrobiellen Probe und/oder dem Nährmedium oder getrennt von diesen auf den Probenfleck aufgebracht werden. Grundsätzlich vorstellbar ist auch, die Reihenfolge des Ablegens und Dispensierens umzudrehen, so dass zuerst ein Tropfen dispensiert wird, in den dann die mikrobielle Probe eingebracht wird.

Offenbart, nicht dem Schutzbereich der beigefügten Patentansprüche unterfallend und der Veranschaulichung dienend ist auch ein Verfahren zur Aufbereitung von Mikroorganismen für eine anschließende massenspektrometrische Messung, das die folgenden Schritte aufweist: (a) Bereitstellen eines flachen Probenträgers, der mehrere Probenflecken umfasst; zum Beispiel ein *MSP 48*/*96 target polished steel BC* von Bruker Daltonik GmbH; (b) Aufbringen von intakten, abseits des Probenträgers gezüchteten und/oder separierten Mikroorganismen in einem Nährmedium-Tropfen auf wenigstens einen der Probenflecken des flachen Probenträgers; vorzugsweise mit einer Nano- oder Mikropipette; die Menge der übertragenen Tropfen kann zwischen 1 und 10 Mikrolitern betragen; (c) Aufbewahren des flachen Probenträgers für eine vorbestimmte Stehzeit, um eine Bildung einer Mikroorganismenablagerung auf dem Probenfleck zuzulassen; vorzugsweise etwa 10 bis 60 Minuten; (d) Entfernen von Restflüssigkeit des Nährmedium-Tropfens nach der vorbestimmten Stehzeit, um die Mikroorganismenablagerung freizulegen; (e) Präparieren des Probenflecks für eine desorbierende Ionisierung; vorzugsweise mit MALDI-Matrixsubstanz; (f) Überführen des Probenträgers in eine Desorptions-Ionenquelle eines Massenspektrometers, Erzeugen von Ionen aus dem präparierten Probenfleck und Aufnehmen wenigstens eines zugehörigen Massenspektrums; und (g) Abgleichen des aufgenommenen Massenspektrums mit einem Referenzdatensatz, um wenigstens eine Eigenschaft der Mikroorganismen zu ermitteln.

Die Erfinder haben festgestellt, dass sich eine Mikroorganismenablagerung auf einer ebenen Fläche schon nach relativ kurzer Stehzeit (bzw. "Ruhezeit") von bis zu einer Stunde derart ausbilden kann, dass sich die dort in einer Art "Biofilm" sedimentierten Mikroorganismen behutsam von Restflüssigkeit befreien und mit einer anschließenden massenspektrometrischen Messung zuverlässig nachweisen lassen. Diese erstaunliche Erkenntnis ausnutzend können die Mikroorganismenzüchtung (bzw. Bebrütung) zwecks Wachstumsförderung und die Präparation für die massenspektrometrische Messung, welche gemäß dem ersten Aspekt der Offenbarung auf dem gleichen massenspektrometrischen Probenträger ausgeführt werden, auf unterschiedlichen Substraten erfolgen. Diese räumliche Trennung eröffnet Anwendungsmöglichkeiten insbesondere in der Automatisierung von Arbeitsabläufen, da sich ein automatisiertes Züchtungsprotokoll in einem klinischen Umfeld unter Umständen leichter in Gefäßen wie zum Beispiel Näpfen einer standardisierten Mikrotiterplatte als auf einer ebenen Fläche wie bei einem MALDI-TOF MS-Träger ausführen lassen könnte. Grundsätzlich gelten die Ausführungen zum Verfahren gemäß dem ersten Aspekt auch für das nicht dem Schutzbereich der beigefügten Patentansprüche unterfallende und der Veranschaulichung dienende Verfahren, sofern sie sich damit in Einklang bringen lassen.

In verschiedenen Ausführungsformen kann der Referenzdatensatz Referenzspektren aufweisen, die einer Bibliothek früher aufgenommener Massenspektren entnommen werden, wobei die wenigstens eine Eigenschaft aus Schritt (g) im Rahmen einer Identifizierung Art oder Unterart der Mikroorganismen umfasst.

In verschiedenen Ausführungsformen können die Mikroorganismen vor dem Aufbringen in Schritt (b) in wenigstens einem Gefäß abseits des flachen Probenträgers in einem flüssigen Nährmedium gezüchtet und von dort auf den Probenfleck übertragen werden; vorzugsweise dauert die Züchtung in einem konditionierten/temperierten Brutschrank etwa 4 bis 24 Stunden. Die Züchtung ist insbesondere für eine Empfindlichkeitstestung der Mikroorganismen nützlich - vorzugsweise jeweils in Anwesenheit und Abwesenheit eines Antibiotikums, wie zuvor geschildert. Für eine Identifizierung oder Bestimmung bestimmter Proteine im Massenspektrum, die auf bestimmte Virulenzfaktoren oder andere mikrobielle Eigenschaften hinweisen können, ist eine Züchtung nicht zwingend erforderlich und braucht daher nicht Teil des unter Schutz gestellten Verfahrens zu sein. Man kann eine bereits existierende Mikroorganismen-Suspension verwenden; zum Beispiel eine, die als Folge anderer Arbeitsschritte im Labor entstanden ist. Als Beispiel sei genannt: eine Suspension von beispielsweise Bakterien wird durch Auflösung der auf Agarplatten vorliegenden Bakterienkolonien in Flüssigkeit hergestellt; vorausgegangen sein könnte eine Züchtung der Bakterien auf einem Festmedium wie Agar, die die Kolonien sprießen lässt (sie könnten bereits seit Tagen in dieser Form vorliegen). Ein weiteres Beispiel: das MALDI Sepsityper^{®} Kit verwendet die Flüssigkeit aus positiven Blutkulturflaschen, welche das Blut septischer Patienten und Flüssigmedium und im positiven Fall angezüchtete Erreger enthalten. Das Kit reichert den Erreger aus dieser positiven Flüssigkeit weiter an durch ein Lyse/Zentrifugations-Verfahren - mit anschließender Proteinextraktion und MALDI-Messung der Proteine. Alternativ lassen sich einige Mikroliter der positiven Blutkulturflüssigkeit auf einen Fleck aufbringen. Auch ohne Bebrütung unter Wärmezufuhr sondern bei Raumtemperatur werden die Mikroorganismen-Zellen (i) sedimentieren und (ii) sich an die Oberfläche des Trägers heften; (iii) ferner werden sich die meisten Spezies sogar bei Raumtemperatur geringfügig vermehren.

In verschiedenen weiteren Ausführungsformen können die gleichen Mikroorganismen in mehreren (externen) Gefäßen gezüchtet werden, und dem flüssigen Nährmedium lässt sich mal eine antimikrobielle Substanz beimengen und mal nicht. Zusätzlich kann dem flüssigen Nährmedium in verschiedenen Gefäßen mit beigemengter antimikrobieller Substanz mal ein Enzym-Hemmer beigemengt werden und mal nicht. Ein Beispiel ist ein β-Laktamase-Hemmer.

In verschiedenen Ausführungsformen kann der Referenzdatensatz ein zeitnah aufgenommenes Massenspektrum eines Probenflecks sein, auf dem sich eine Mikroorganismenablagerung befindet, die aus einem flüssigen Nährmedium ohne antimikrobielle Substanz oder Enzym-Hemmer hervorgegangen ist, und die wenigstens eine Eigenschaft aus Schritt (g) kann im Rahmen einer Charakterisierung eine Empfindlichkeit der Mikroorganismen gegenüber der antimikrobiellen Substanz bzw. einer Kombination aus antimikrobieller Substanz und Enzym-Hemmer umfassen. In einer besonderen Variante lassen sich die gleichen Mikroorganismen in verschiedenen (externen) Gefäßen mit flüssigem Nährmedium bei jeweils verschiedenen Konzentrationen züchten, und die wenigstens eine Eigenschaft aus Schritt (g) kann im Rahmen einer Charakterisierung eine minimale Hemmkonzentration der antimikrobiellen Substanz bezüglich der Mikroorganismen umfassen.

Vorzugsweise wird die wenigstens eine Eigenschaft in Schritt (g) aus einem Unterschied im Mikroorganismenwachstum abgeleitet, das sich beispielweise in einem Unterschied zwischen verlässlicher und fehlgeschlagener Identifizierung mit dem MALDI Biotyper^{®}-Algorithmus manifestieren kann.

Die Mikroorganismen lassen sich zu Beginn einer Züchtung derart bemessen, dass eine Menge leicht unterhalb der Nachweisgrenze der massenspektrometrischen Messung liegt; beispielsweise bei etwa 10⁵ cfu pro Milliliter Nährmedium, insbesondere führend zu einer Konzentration von mindestens etwa 100 Mikroorganismen pro Fleck.

In verschiedenen Ausführungsformen kann das Entfernen von Restflüssigkeit vom flachen Probenträger in Schritt (d) ein Abtupfen eines Tropfenüberstandes mittels eines saugfähigen Materials oder ein Abpipettieren umfassen.

Das Präparieren in Schritt (e) kann eine vorbereitende Extraktion mikrobieller Proteine/Peptide aus der Mikroorganismenablagerung auf dem flachen Probenträger und/oder ein Waschen der Mikroorganismenablagerung und/oder eine Einbettung der Mikroorganismenablagerung in eine Laserlicht-absorbierende Matrixsubstanz, um anschließend in Schritt (f) per matrix-unterstützter Laserdesorption (MALDI) ionisiert zu werden, umfassen.

Die Massenspektren in Schritt (f) werden bevorzugt Flugzeit-dispersiv (in einem Flugzeit-Massenspektrometer) aufgenommen.

In verschiedenen Ausführungsformen lassen sich die Mikroorganismen (i) als Suspension im flüssigen Nährmedium in das (externe) Gefäß dispensieren oder (ii) zuerst in Zellform in das (externe) Gefäß einbringen, wonach flüssiges Nährmedium eingefüllt wird; die Nährmedienmenge kann zum Beispiel zwischen 10 und 100 Mikrolitern betragen.

In verschiedenen Ausführungsformen kann ein Napf (oder mehrere Näpfe) in einer Mikrotiterplatte als (externes) Gefäß (bzw. externe Gefäße) für die Züchtung verwendet werden. Alternativ lässt sich die Probenträgerplatte aus Schritt (a) in einen ersten flachen Abschnitt mit ebenen Probenflecken und einen zweiten Abschnitt mit Vertiefungen an der Oberfläche unterteilen, wobei die Vertiefungen als (externe) Gefäße verwendet werden (abseits des ersten flachen Abschnitts) und die darin gezüchteten, intakten Mikroorganismen in Schritt (b) von dort auf ebene Probenflecken im ersten flachen Abschnitt übertragen werden.

### Kurze Beschreibung der Abbildungen

Zum besseren Verständnis der Erfindung wird auf die folgenden Abbildungen verwiesen. Die Elemente in den Abbildungen sind nicht unbedingt maßstabsgetreu dargestellt, sondern sollen in erster Linie die Prinzipien der Erfindung (größtenteils schematisch) veranschaulichen. In den Abbildungen sind einander entsprechende Elemente in den verschiedenen Ansichten durch gleiche Bezugszeichen gekennzeichnet.

Abbildung 1 zeigt schematisch ein Ausführungsbeispiel für ein Massenspektrometer 10 mit linear-axialer Flugstrecke 2, die an einem Detektor 4 endet, und vorgelagerter Ionenquelle 6 für die matrix-unterstützte Laserdesorption (MALDI), wie sie bei einer massenspektrometrischen Untersuchung mikrobieller Zellen häufig zum Einsatz kommen.

Abbildung 2A bis Abbildung 2G zeigen schematisch und beispielhaft ein Aufbereitungsverfahren für Mikroorganismen in einer mikrobiellen Probe zwecks Identifizierung.

Abbildung 3A bis Abbildung 3H veranschaulichen schematisch einen möglichen Verfahrensablauf für eine Resistenz/Empfindlichkeitstestung einer mikrobiellen Probe.

Abbildung 4A und Abbildung 4B präsentieren Ergebnisse einer Resistenz/Empfindlichkeitstestung gemäß den Prinzipien der hier vorgestellten Verfahren.

Abbildung 5A bis Abbildung 5I zeigen schematisch und beispielhaft ein Aufbereitungsverfahren für Mikroorganismen, das nicht dem Schutzbereich der beigefügten Patentansprüche unterfällt und der Veranschaulichung dient.

Abbildung 6 illustriert ein Ausführungsbeispiel eines kombinierten Probenträgers mit Abschnitten, die einerseits flach sind und daher als massenspektrometrischer Probenträger (und ggf. die Präparation darauf) verwendet werden können und andererseits eingebaute Gefäße für die Züchtung/Bebrütung von Mikroorganismen in flüssigen Nährmedien aufweisen.

### Detaillierte Beschreibung

Während die Erfindung anhand einer Anzahl von Ausführungsformen dargestellt und erläutert wurde, werden Fachleute auf dem Gebiet anerkennen, dass verschiedene Änderungen in Form und Detail daran vorgenommen werden können, ohne vom Umfang der in den beigefügten Patentansprüchen definierten technischen Lehre abzuweichen.

Gemäß einem ersten bevorzugten Aspekt der Offenbarung wurde überraschenderweise festgestellt, dass die Aufbereitung einer lebendigen, mikrobiellen Probe direkt auf einem massenspektrometrischen Probenträger allein für die Anzucht von Mikroorganismen eingesetzt werden kann, um eine für den massenspektrometrischen Nachweis ausreichende Mikroorganismenzahl auf den Probenflecken zu erzeugen. Der Nährmedium-Tropfen, in dem die lebendigen, mikrobiellen Zellen suspendiert oder eingetaucht sind, fungiert in dieser einfachen, aber gleichwohl überraschend effizienten Ausführungsform gleichsam als Brutreaktor.

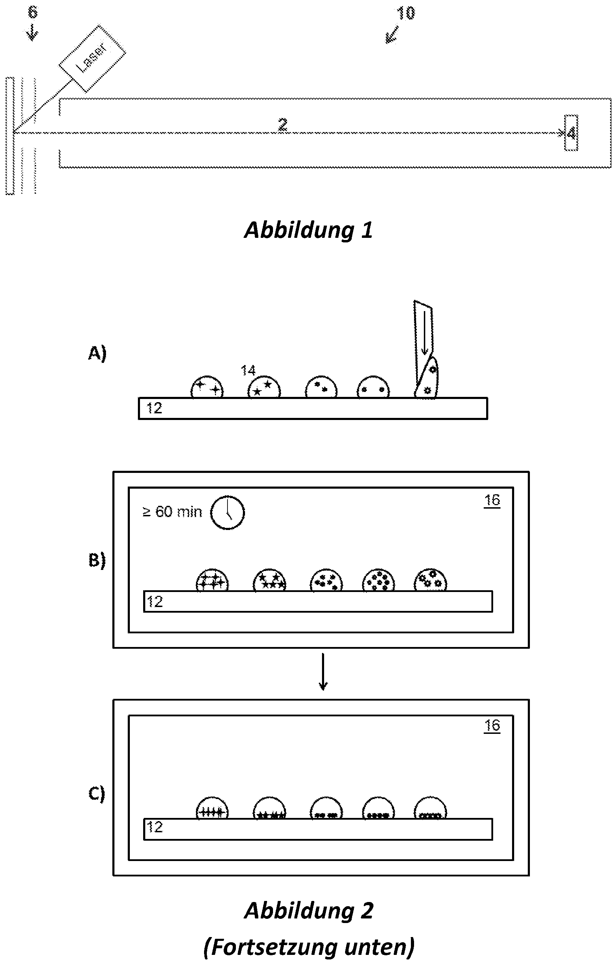

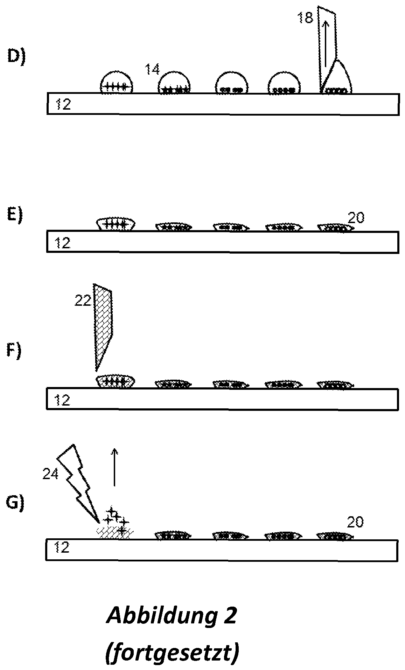

Abbildung 2A bis **Abbildung 2G** illustrieren schematisch ein solches Kultivierungsverfahren in einem Tropfen direkt auf dem Probenträger mit anschließender massenspektrometrischer Messung.

Ein flacher Probenträger 12 wird an unterschiedlichen Stellen ("Flecken") mit Tropfen 14 einer Mikroorganismen-Suspension belegt, wobei die Flüssigkeit ein Nährmedium wie zum Beispiel eine Kationen-adjustierte Mueller-Hinton-Bouillon oder eine Iso-Sensitest-Bouillon enthält. Die Tropfen 14 können je nach Anforderung ein Volumen von 1-10 Mikroliter aufweisen, zum Beispiel 2, 4, 6 oder auch 8 Mikroliter. Die Probenflecken können auf der Trägeroberfläche ausgezeichnet sein, oder aber auf Stellen in genügendem Abstand auf eine ansonsten merkmalsfreie Oberfläche des Trägers 12 aufgebracht werden. Als Träger 12 lässt sich beispielsweise eine AnchorChip-Platte mit 96 markierten Flecken verwenden (Bruker Daltonik GmbH, Bremen, Deutschland). Alternativ können die Probenflecken auch durch den aufgebrachten Nährmedium-Tropfen 14 definiert sein, welcher bei einer hinreichend hydrophoben Oberfläche des Probenträgers 12 nicht zerlaufen kann. **Abbildung 2A****.**

Der in diesem Beispiel mit fünf Tropfen 14 jeweils unterschiedlichen Mikroorganismeninhalts belegte Träger 12 wird in einen Bebrütungsraum 16 verbracht, wo er unter definierter, kontrollierter Atmosphäre von z.B. 36°C und nahezu 100% relativer Luftfeuchte für bis zu etwa 18 Stunden gehalten werden kann. Es sei hier erwähnt, dass das Aufbringen der mikrobiellen Proben natürlich auch bereits in dem Bebrütungsraum 16 erfolgen kann; die zugehörigen Verfahrensschritte des Aufbringens auf den Probenträger 12 und Verbringen des Probenträgers 12 in den Bebrütungsraum 16 können also in jeder denkbaren Ausführungsform der in dieser Offenbarung beschriebenen Verfahrensabläufe die Reihenfolge tauschen.

Die Feuchte im Bebrütungsraum 16 lässt sich beispielsweise mit Natriumchlorid-Lösung einstellen. In der Zeit im Bebrütungsraum 16 können die Mikroorganismen sich unter Verstoffwechslung des Nährmediums in den Tropfen 14 vermehren. Die Vermehrung kann dadurch sichtbar werden, dass sich das anfangs klare Nährmedium im Tropfen 14 nach einigen Stunden eintrübt (nicht dargestellt). Allerdings ist die Eintrübung kein zwingendes Erfordernis für ein massenspektrometrisch nachweisbares Mikroorganismenwachstum. Sie kann jedoch bei hinreichend langen Bebrütungszeiten als visuelle Prozesskontrollmarke dienen. **Abbildung 2B****.** Die Erfinder haben überraschender Weise beobachtet, dass sich gleichzeitig die vermehrten Mikroorganismen in ausreichender Menge an der Grenzfläche zwischen Tropfen 14 und Trägeroberfläche absetzen, was die nachfolgende Flüssigkeits-Abreicherung (Entfeuchtung) bzw. Trocknung stark erleichtert. **Abbildung 2C****.**

Die Restflüssigkeit im Tropfenüberstand lässt sich in einer Variante mittels einer Mikro-oder Nanopipette 18 vorsichtig absaugen, bis die sedimentierten Klumpen mikrobieller Zellen 20 auf der Trägeroberfläche von Flüssigkeit nahezu freigelegt sind. Abbildung 2D-2E. Auf Grund des zuvor beschriebenen Sedimentierungsverhaltens verbleiben durch diese Art der Entfernung von Restflüssigkeit des Nährmediums überraschender Weise ausreichend Mikroorganismen auf den Probenflecken, so dass die Grundlage für die Erzeugung nachweisbarer Massensignale im Detektor des Massenspektrometers erhalten bleibt.

Anschließend kann die freigelegte Mikroorganismenablagerung 20 auf den Probenflecken mit einer Matrixsubstanz für die Ionisierung mittels matrix-unterstützter Laserdesorption direkt auf dem Probenträger 12 belegt werden (Pipettenspitze 22 mit Kachelschraffur). **Abbildung 2F.** Zusätzlich können davor noch weitere Bearbeitungsschritte wie die Zugabe von Substanzen zur Protein/Peptid-Extraktion und/oder ein Wasch-Schritt eingeschoben werden (nicht dargestellt), die dem Fachmann wohlbekannt sind. In der Ionenquelle des Massenspektrometers werden die derart präparierten Probenflecken mit einem Laser beschossen 24, wodurch Ionen aus dem sedimentierten und präparierten Mikroorganismenfilm 20 auf dem Probenfleck erzeugt und dem angeschlossenen Massenanalysator zur Messung zugeführt werden. **Abbildung 2G****.**

Die Art oder Unterart des gezüchteten Mikroorganismus kann aus den spezifischen Massensignalen in den aufgenommenen Massenspektren mittels bekannter Auswerte-Algorithmen wie dem MALDI Biotyper^{®} (Bruker Daltonik GmbH, Bremen, Deutschland) durch Vergleich mit Referenzspektren aus einer Spektren-Datenbank mit hoher Verlässlichkeit abgeleitet werden. Die Vorgehensweise bei einer solchen Auswertung ist dem Fachmann bekannt und braucht hier nicht näher geschildert zu werden.

Neben der zuvor erläuterten reinen Identifizierungsmessung nach Mikroorganismenzüchtung direkt auf dem massenspektrometrischen Probenträger stellt die vorliegende Offenbarung auch Verfahren und Testkits für die Verarbeitung der mikrobiellen Proben für eine Resistenz/Empfindlichkeitstestung bereit, wie im Folgenden erläutert wird.

Die üblichen Geräte für die Empfindlichkeitstestung testen in der Regel eine große Anzahl der Antibiotika gleichzeitig (zum Beispiel 12 bis 18), die in standardisierten, sogenannten "Panels" enthalten sind. Die Ergebnisse liegen in der Regel nicht am gleichen Arbeitstag vor, da sie längere Bebrütungs- und Reaktionszeiten benötigen; übliche Inkubationszeit: 10-24 Stunden (sogenannte "Übernacht-Bebrütung"). In konkreten klinischen Situationen kann es ausreichend sein, nur ein Antibiotikum oder einige wenige Antibiotika zu testen, die bei einem bestimmten Patienten und bei seiner Erkrankung spezifisch in Frage kommen, oder die bei einem bestimmten Patienten bereits verabreicht worden sind. Es kann aber dringend geboten sein, die Wirksamkeit zu überprüfen, wenn beispielsweise keine klinische Besserung unter Antibiotikagabe zu beobachten ist. Dafür ist es sehr wichtig, insbesondere bei lebensbedrohlichen Infektionen wie zum Beispiel der Sepsis, das Ergebnis einer solchen Testung dem behandelnden Arzt nicht am nächsten Tag sondern bereits innerhalb einer deutlich kürzeren Zeit von beispielsweise wenigen Stunden als therapeutische Entscheidungshilfe zur Verfügung zu stellen.

Aus diesem Grund fokussiert sich diese Beschreibung insbesondere auf Verfahren und Vorrichtungen (Testkits, Verbrauchsmaterialien und sonstige Utensilien) für die Durchführung einzelner Schnellteste, d.h. Testung eines bestimmten Antibiotikums gegenüber einem bestimmten Mikroorganismus bzw. gegenüber Gruppen von Mikroorganismen. Dies schließt nicht aus, dass gleiche oder ähnliche bzw. abgeleitete Prinzipien, gegebenenfalls in Anpassung an die gleichzeitige Testung mehrerer Antibiotika, für eine gleichzeitige oder zeitnahe Testung einer großen Anzahl der Antibiotika angewendet werden können.

Eines der Merkmale der beschriebenen Methode gemäß dem ersten bevorzugten Aspekt ist die Durchführung der Resistenz/Empfindlichkeitstestung, zumindest des Großteils der erforderlichen Arbeitsschritte, direkt auf einem massenspektrometrischen Probenträger wie einem MALDI-TOF MS-Träger, d.h. einer dazu geeigneten flachen und leitfähigen Platte aus zum Beispiel poliertem Stahl oder Keramik, die bei der Messung in der Ionenquelle eines Massenspektrometers als Substrat der Ionisierung dient. Bei der beschriebenen Methode erfolgt die Empfindlichkeitstestung wachstumsbasiert (kulturbasiert), d.h. die Methode stellt eine phänotypische Empfindlichkeitstestung dar und ist damit unabhängig von den zu Grunde liegenden Resistenzmechanismen (sofern vorhanden).

Das Wachstum der Mikroorganismen mit und ohne Antibiotikum (letzteres kennzeichnet die Wachstumskontrolle) kann dabei ebenfalls direkt auf einem MALDI-TOF MS-Träger stattfinden, auf dem anschließend auch die Messung erfolgt. Dies unterscheidet die beschriebene Methode gemäß dem ersten bevorzugten Aspekt grundsätzlich von den bisherigen MALDI-TOF MSbasierten Methoden, bei denen die Mikroorganismen außerhalb des MALDI-TOF MS-Trägers gezüchtet werden. Bei solchen Methoden erfolgt die Anzucht mit bzw. ohne Antibiotikum (für die Wachstumskontrolle) zuerst in Kulturgefäßen oder Näpfen von Mikrotiterplatten, wonach eine Isolierung der Mikroorganismen bzw. der mikrobiellen Proteine in diesen Gefäßen oder Näpfen stattfindet, welche anschließend auf einen MALDI-TOF MS-Träger übertragen und danach gemessen werden. Dies erfordert allerdings eine Reihe arbeitsaufwändiger, händischer Schritte, was in einer schlechten Integrierbarkeit derartiger, bisheriger Verfahren in die Routinediagnostik resultiert. Die vorgeschlagene Methode gemäß dem ersten Aspekt ermöglicht dagegen eine sehr viel einfachere und schnellere Verarbeitung der Proben.

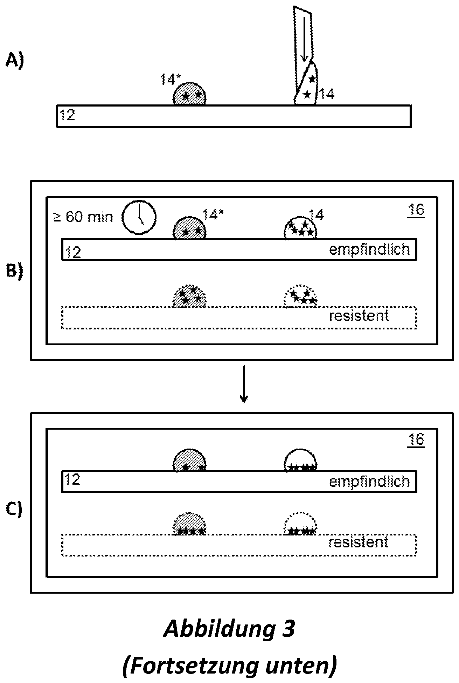

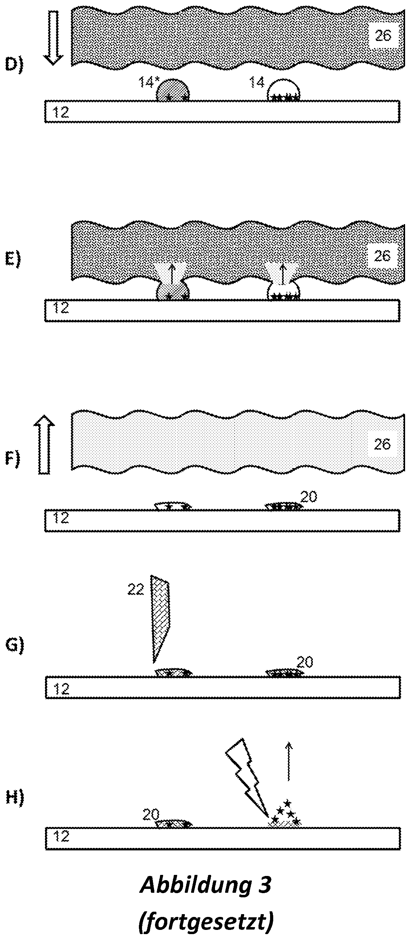

Ähnlich wie zuvor in Bezug auf Abbildung 2A bis **Abbildung 2G** geschildert, begleiten Abbildung 3A bis **Abbildung 3H** die hier beschriebenen Arbeitsschritte beispielhaft und dienen der schematischen Veranschaulichung ihrer möglichen Bedeutung. Praktiker auf dem Gebiet werden jedoch anerkennen, dass sich bestimmte Arbeitsschritte in abgewandelter Form ausführen lassen. Der Fachmann wird den hier vorgeschlagenen Arbeitsablauf als Orientierungshilfe verstehen und gegebenenfalls davon im Rahmen seines Routinekönnens und Routinewissens abweichen, sofern es ihm geboten oder nützlich scheint.

Das Antibiotikum (zum Beispiel eine Antibiotikum-Lösung in einem flüssigen Nährmedium) kann mit einer Mikroorganismen-Suspension entweder in einem Kulturgefäß oder direkt auf einem Fleck eines MALDI-TOF MS-Trägers 12 gemischt werden (schraffierter Tropfen 14*). Zusätzlich wird in der Regel eine Wachstumskontrolle auf einem anderen Bereich des MALDI-TOF MS-Trägers mitgeführt, d.h. eine Kultivierung der Mikroorganismen-Suspension in einem Nährmedium ohne Zugabe eines Antibiotikums (unschraffierter Tropfen 14). Es wird vorzugsweise eine sehr kleine Menge der Suspension auf die Flecken appliziert; zum Beispiel 1-10 Mikroliter. Somit findet die Empfindlichkeitstestung in Mikrotröpfchen 14, 14* statt, vorzugsweise von etwa 4-8 Mikroliter Volumen. Die Verwendung von noch kleineren Volumina ist grundsätzlich auch möglich, beispielsweise in Form von Nanotröpfchen. Die initiale Mikroorganismen-Konzentration in den Tropfen 14, 14* kann leicht unterhalb der Nachweisgrenze eines konventionellen MALDI-Flugzeit-Massenspektrometers mit linearer Flugstrecke liegen und somit zum Beispiel etwa 5×10⁵ cfu pro Milliliter betragen (cfu - *colony forming unit*; Kolonie-bildende Einheit). **Abbildung 3A****.**

In dem gezeigten Beispiel wird der MALDI-TOF MS-Träger 12 mit dem Testansatz anschließend in einer sogenannten "feuchten Kammer" 16 (Feuchtkammer) in einem Inkubator bei hoher Luftfeuchtigkeit bebrütet. Die Feuchtkammer 16 dient dem Zweck, dass die Tröpfchen 14, 14* während der Inkubation nicht vorzeitig verdunsten, und kann als eine Schachtel mit Deckel, zum Beispiel aus Kunststoff, ausgeführt sein, in die sich der MALDI-TOF MS-Träger mühelos hineinlegen lässt. Diese Feuchtkammer 16 kann ähnlich wie die handelsüblichen Transport-oder Aufbewahrungsbehälter für kommerzielle MALDI-TOF MS-Träger (Bruker Daltonik GmbH, Bremen, Deutschland) ausgeführt werden und wird vorzugsweise derart konstruiert, dass hierbei der MALDI-TOF MS-Träger 12 tief genug darin platziert werden kann, damit der Deckel nicht in Kontakt mit den Tröpfchen 14, 14* auf der Oberfläche des MALDI-TOF MS-Trägers 12 kommt. In die Feuchtkammer 16 kann eine kleine Menge Flüssigkeit eingefüllt werden, beispielsweise 0,1 bis 5 Milliliter Wasser oder NaCl-Lösung, um die Atmosphäre in der Kammer 16 mit Feuchtigkeit anzureichern und dadurch eine hohe Luftfeuchte einzustellen (nahe 100%), so dass die Verdunstung der Nährmedium-Tröpfchen 14, 14* selbst verhindert wird. **Abbildung 3B****.**

In den Tröpfchen 14, 14* wird bei Inkubation schnell eine hohe Konzentration der Mikroorganismen in einem kleinem Flüssigkeitsvolumen erreicht (sofern das Wachstum nicht durch antimikrobielle Substanzen gehemmt wird). Nach ausreichender Zeit lässt sich die Feuchtkammer 16 mit dem MALDI-TOF MS-Träger 12 aus dem Bebrütungsschrank (nicht gezeigt) entnehmen. Anschließend wird der MALDI-TOF MS-Träger 12 aus der Feuchtkammer 16 entfernt und die sich darauf befindenden Tröpfchen 14, 14* werden getrocknet. Die Trocknung kann passiv zum Beispiel an Luft erfolgen oder beispielsweise durch einen aktiv erzeugten Luftstrom, Wärmeeinwirkung, eine Kombination daraus oder andere Verfahren beschleunigt werden. Auf Grund des sehr kleinen Flüssigkeitsvolumens eines Tröpfchens 14, 14* (Nanoliter bis Mikroliter) erfolgt die Trocknung sehr schnell. Diese einfache Trocknung der Tröpfchen (beispielsweise mit Heißluft) kann jedoch den Nachteil haben, dass sich neben den mikrobiellen Zellen auch Proteine und andere Bestandteile des flüssigen Nährmediums auf den Flecken des MALDI-TOF MS-Trägers 12 anreichern und die MALDI-TOF MS-Messung stören. Dieses potentielle Problem lässt sich dadurch beheben, dass die mikrobiellen Zellen vom Nährmedium direkt auf dem MALDI-TOF MS-Träger 12 separiert werden.

In beiden Fällen des Trocknens und der Separation ist das Ziel, die Restflüssigkeit des Nährmedium-Tropfens 14, 14* weitgehend zu entfernen, um den Probenfleck für die anschließende Präparation vorzubereiten. Wie zuvor angedeutet haben die Erfinder während ihrer Untersuchungen festgestellt, dass die mikrobiellen Zellen die Neigung aufzuweisen scheinen, während der Inkubation, die einige Stunden dauern kann, zu sedimentieren, um sich dann überwiegend unmittelbar an der Oberfläche des MALDI-TOF MS-Trägers 12 anzusammeln. In einem bestimmten Ausmaß adhärieren ("kleben") die Zellen sogar an die Oberfläche des Trägers 12 und bilden eine Art "Mikroorganismus-Biofilm", wohingegen flüssige Bestandteile einen darüber angeordneten "Überstand" bilden. Ohne eine ausgereifte wissenschaftliche Erklärung für dieses Verhalten während des Mikroorganismenwachstums in einem Tropfen auf einer flachen Platte geben zu wollen, wird vermutet, dass physikalische Wechselwirkungen zwischen der Plattenoberfläche und den Mikroorganismus-Zellen sowie Adhäsionsprozesse durch die biochemischen und biophysikalischen Eigenschaften der Mikroorganismen-Zelloberfläche für die bevorzugte Anlagerung verantwortlich sind. **Abbildung 3C****.**

Diesen neuen Befund ausnutzend kann das überstehende Flüssignährmedium (Restflüssigkeit) von auf dem MALDI-TOF MS-Träger 12 befindlichen Tröpfchen 14, 14* abpipettiert werden, wie es in Bezug auf **Abbildung 2D** in anderem Zusammenhang bereits geschildert worden ist. Alternativ kann es auch einfach abgetupft werden, um die Mikroorganismenablagerung von Flüssigkeit freizulegen. Dafür lassen sich beispielsweise saugfähige, fusselarme Wischtücher 26 verwenden, wie sie in biologischen/chemischen Laboratorien üblich sind; beispielsweise KimWipes^{™}. Die Separation erfolgt dabei sofort und kann unter anderem durch Kapillareffekt erklärt werden. Die Separation lässt sich durch manuelles Abtupfen mithilfe zum Beispiel eines gefalteten Tuchs (als da wären Saugpapier, Löschblatt, weiche Tücher für die Reinigung empfindlicher Oberflächen) oder mithilfe einer speziellen Vorrichtung ausführen. Eine solche Vorrichtung kann zum Beispiel eine Platte oder ein "Pad" aus einem saugfähigen Material aufweisen, welche für ein gleichmäßiges, schnelles und standardisiertes Abtupfen insbesondere einfach über dem MALDI-TOF MS-Träger 12 in einem Abstand angeordnet wird, welcher die Herstellung eines Fluidkontakts mit den Tropfen 14, 14* erlaubt, und nach einer relativ kurzen Aufsaug-Zeit von einigen Sekunden wieder abgenommen wird. **Abbildungen 3D-3F.**

In einer alternativen Ausführungsform des Abtupfens lässt sich der Kontakt zwischen Tropfen und saugfähigem Gewebe nicht in vertikaler Richtung (senkrecht zur Probenträgeroberfläche wie in **Abbildungen 3D-3F** gezeigt) herstellen, sondern am seitlichen Rand des Tropfens bzw. der Tropfen nahe der Probenträgeroberfläche. Auf diese Weise lässt sich sicherstellen, dass (i) die Restflüssigkeit des Nährmediums schneller und vollständiger aufgenommen wird und (ii) die Zellen, die sich bevorzugt in der Mitte des Flecks an der Oberfläche ansammeln, von dem saugfähigen Gewebe in jedem Fall unberührt bleiben, so dass die Gefahr einer unabsichtlichen Zellentfernung gesenkt wird. Diese Abwandlung der Flüssigkeitsaufnahme kann den Untergrund in den Massenspektren weiter verringern und dadurch die Güte der Messungen noch besser machen.

Auch in diesen vorstehend erläuterten Varianten der Entfernung von Restflüssigkeit resultiert auf dem entsprechenden Probenfleck eine weitgehend entfeuchtete (oder Restflüssigkeits-abgereicherte, freigelegte) Mikroorganismenablagerung 20 mit wenigen Überbleibseln des potentiell störenden Nährmediums, die als Grundlage der anschließenden massenspektrometrischen Messung dient.

Die hier beschriebene Separation der Zellen vom Flüssignährmedium mittels Abtupfen oder Abpipettieren ergibt sehr effektive Messungen mit hoher Qualität der MS-Spektren. Ein weiterer Vorteil dieser Separation im Vergleich zur (passiven) Trocknung der Tropfen ist, dass die Separation extrem schnell geschieht (sofort oder augenblicklich), was einen deutlichen zeitlichen Vorteil liefert und eine direkte Weiterverarbeitung der Proben ermöglicht. Gleichwohl kann das Abtupfen in bestimmten Ausführungsformen von einem geheizten, trocknenden Luftstrom begleitet werden, um die Restflüssigkeit noch vollständiger abzureichern.

Das beschriebene Verfahren ist ähnlich effektiv zur Abtrennung von Zellen aus einem Flüssigmedium wie Zentrifugation mit anschließender Entfernung des Überstandes, ist aber direkt auf einem MALDI-TOF MS-Träger und ohne zeitlichen Aufwand möglich. Der Separationseffekt kann noch verstärkt werden - zum Beispiel durch Verwendung von speziellen MALDI-TOF MS-Trägern, wie beispielsweise Anker-Trägern (*AnchorChip,* Bruker Daltonik GmbH, Bremen) oder auch MALDI-TOF MS-Träger mit einzelnen Probenflecken in Form eines abgeflachten Konus. Dies könnte den Zell-Sedimentierungseffekt durch flache aber leicht konische Vertiefungen verstärken. Auch andere Verfahren der Waschung von flüssigen Proben direkt auf einem Träger können Anwendung finden.

Um die Bildung einer Mikroorganismenablagerung (Adhäsion) auf der Probenträgeroberfläche während der Wachstumsphase zu verbessern, können die Flecken-Oberflächen mit unterschiedlichen Adhäsions-verbessernden Substanzen beschichtet werden; zum Beispiel Proteinen oder Zuckern. Diese Substanzen werden vorzugsweise so gewählt, dass sie die Messung und/oder den Abgleich der mikrobiellen Massenspektren mit Referenzdatensätzen nicht stören. Dies lässt sich beispielsweise dadurch erreichen, dass sich die Massensignale dieser Substanzen außerhalb des auszuwertenden Massenbereiches befinden, welcher üblicherweise zwischen m/z 2.000 und m/z 20.000, beispielsweise zwischen m/z 3.000 und m/z 15.000, liegt. Alternativ können als Adhäsions-steigernde Substanzen auch solche Substanzen (zum Beispiel Proteine) gewählt werden, die gleichzeitig als Standard-Substanzen eingesetzt werden; d.h. als Marker für die gute Qualität der Messungen und/oder als Intensitäts-Marker. In diesem Fall können sich die Massensignale dieser Standard-Substanzen in dem auszuwertenden Massenbereich befinden. Ferner können Materialien mit verbesserten Adhäsions-Eigenschaften und/oder verbesserter Oberflächenbeschaffenheit von vornherein als Materialien für die Herstellung der MALDI-TOF MS-Träger verwendet werden.

Nach der Trocknung oder Separation der Tröpfchen von Flüssignährmedium auf den entsprechenden Flecken werden die Flecken zum Beispiel mit einer Matrix für MALDI-TOF MS-Untersuchungen bedeckt (Pipettenspitze 22 mit Kachelschraffur), gefolgt vom Einbringen des Trägers in das MALDI-TOF MS-Gerät und der Messung der mikrobiellen Biomoleküle, beispielsweise Proteine oder Peptide, wie zuvor bereits erläutert. **Abbildungen 3G-3H.**

Vor oder gleichzeitig mit der Applikation der Matrixsubstanz kann für die bessere Extraktion der mikrobiellen Proteine die Zugabe von unterschiedlichen Substanzen erfolgen, die die Messung begünstigen (nicht gezeigt), zum Beispiel von Ameisensäure oder Acetonitril. Auch Tropfen deionisierten Wassers können als Waschtropfen auf die Mikroorganismenablagerung aufgebracht und wieder abgenommen werden, um Salze zu entfernen. Nach der Messung lassen sich die Ergebnisse entsprechend der Algorithmen auswerten, die weiter unten näher erläutert werden. Dabei wird das Wachstum der Mikroorganismen in Anwesenheit der Antibiotika beurteilt und bewertet. Grundsätzliches Prinzip ist, dass das Wachstum der empfindlichen Mikroorganismen in Anwesenheit der Antibiotika gehemmt wird, während die resistenten Mikroorganismen in der Lage sind, trotz Antibiotikum zu wachsen. Eine Mitführung der Wachstumskontrolle, d.h. eine Testung der Mikroorganismen-Suspension ohne Antibiotikum auf dem gleichem MS-Probenträger, kann hilfreich für die Auswertung und für die entsprechenden Auswertungs-Algorithmen sein. **Abbildung 3B** und **Abbildung 3C** zeigen schematisch das Wachstums- bzw. Sedimentationsverhalten bei Empfindlichkeit (durchgezogene Kontur) und Resistenz (gestrichelte Kontur) in Gegenüberstellung.

In einer Variante des Verfahrens kann die Kultivierung der Mikroorganismen mit bzw. ohne Antibiotikum auch auf einer Komposit-Mikrotiterplatte erfolgen, wo ein flacher, ebener massenspektrometrischer Probenträger wie ein MALDI-TOF MS-Träger den Boden bildet und zusammen mit einem abnehmbaren Aufsatz, der Durchgangsbohrungen enthält, ein Raster aus Näpfen bereitstellt, wie in der Patentanmeldung CA 2 467 131 A1 beschrieben (dort Abbildung 10). Die bereitgestellten Reaktionsgefäße oder Näpfe können (beispielsweise als Testkit) bereits vor der Zugabe einer Mikroorganismus-Suspension Antibiotika enthalten, zum Beispiel in Form einer Lösung, eines Pulvers, oder in einer lyophilisierten Form. Nach ausreichender Bebrütungszeit mit einhergehendem oder ggf. ausbleibendem Mikroorganismenwachstum wird die Restflüssigkeit der Tröpfchen abgenommen, beispielsweise durch Eintrocknen, und der Aufsatz wird von der MALDI-TOF MS-Platte entfernt. Anschließend können eine MALDI-Matrix-Präparation, wie oben beschrieben, und eine MALDI-TOF MS-Messung folgen.

In einer weiteren Ausführungsform lässt sich der MALDI-TOF MS-Träger nicht in einem getrennten Brutschrank bebrüten, sondern die Inkubations-Funktion kann direkt in das MALDI-TOF Massenspektrometer bzw. in das Gesamtsystem integriert werden, zum Beispiel in Form einer Bebrütungseinheit oder eines Bebrütungsmoduls. Das ermöglicht eine Automatisierung und weitere Reduktion der erforderlichen manuellen Verarbeitungsschritte. Eine weitere Ausführungsform sieht die Integration einer Wärmevorrichtung direkt in die Feuchtkammer vor, so dass die Feuchtkammer die Funktion eines Brutschranks übernehmen kann und das Bereitstellen eines separaten Brutschranks überflüssig wird.

Die Verwendung von massenspektrometrischen Probenträgern, die bereits mit Antibiotika auf den Flecken in Form eines trockenen Pulvers oder in einer anderen Form vorpräpariert wurden, kann die Empfindlichkeitstestung für den Anwender zusätzlich erleichtern.

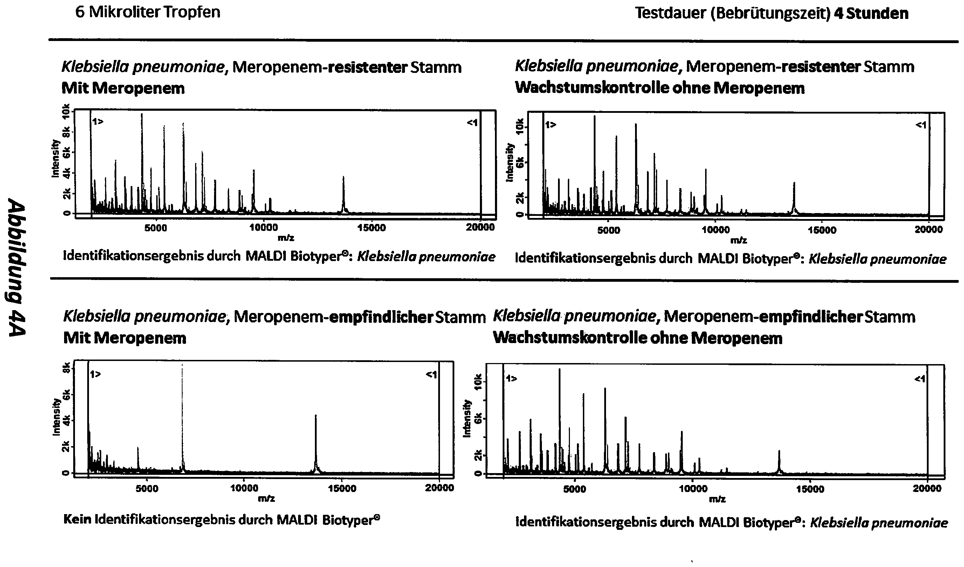

**Abbildung 4A** zeigt Ergebnisse einer Resistenz/Empfindlichkeitstestung am Beispiel des fakultativ anaeroben, gramnegativen Stäbchenbakteriums *Klebsiella pneumoniae* gegenüber dem β-Laktam-Antibiotikum Meropenem aus der Gruppe der Carbapeneme. Die Probenaufbereitung erfolgte direkt auf dem Probenträger, wie schematisch in den **Abbildungen 3A-3H** beschrieben. Das Volumen der dispensierten Tropfen betrug sechs Mikroliter; die Konzentration des Antibiotikums 2 Mikrogramm pro Milliliter; und die Aufenthaltsdauer im entsprechend konditionierten Bebrütungsraum vier Stunden. MALDI-Flugzeit-Massenspektren wurden mit dem Software-Modul des kommerziellen Produkts MALDI Biotyper^{®} ausgewertet. Es wurden je ein Meropenem-resistenter Stamm (Massenspektren oben) und ein Meropenem-empfindlicher Stamm (Massenspektren unten) des Bakteriums getestet. Die Wachstumskontrolle ohne Zugabe des Antibiotikums, die auf der gleichen MALDI-Probenträgerplatte präpariert wurde, ist jeweils im rechten Spektrum dargestellt.

Es ist deutlich erkennbar, dass sich im Falle des resistenten Stammes die Signaturen spezifischer Massensignale in den beiden oberen Spektren kaum unterscheiden. Daraus kann auf Resistenz geschlossen werden, da das Bakterienwachstum durch Gegenwart des Meropenems offensichtlich nicht gehemmt und eine verlässliche Identifizierung der Art möglich ist. Im Falle des empfindlichen Stammes hingegen sind spezifische Massensignal-Signaturen lediglich im Spektrum der Wachstumskontrolle erkennbar (unten rechts). In Gegenwart von Meropenem (Spektrum unten links) können sich die *Klebsiella*-Zellen jedoch offensichtlich nicht (oder kaum) vermehren. Die vereinzelt hervorstehenden Massensignale im Spektrum unten links gehören zu einer Referenzsubstanz, die dem Nährmedium-Tropfen zwecks Mikroorganismen-Quantifizierung beigemengt wurde, bleiben bei der hier konkret beschriebenen Untersuchung jedoch unberücksichtigt. Die Auswerte-Software vermag unter diesen Bedingungen fehlenden Wachstums die Art des Mikroorganismus mangels Datengrundlage nicht zu bestimmen; insbesondere wenn die Ausgangsmenge an mikrobieller Biomasse unterhalb der massenspektrometrischen Nachweisgrenze liegt. Dies erlaubt den Schluss, dass die *Klebsiellen* dieses Stammes auf diese spezifische antimikrobielle Substanz empfindlich reagieren.

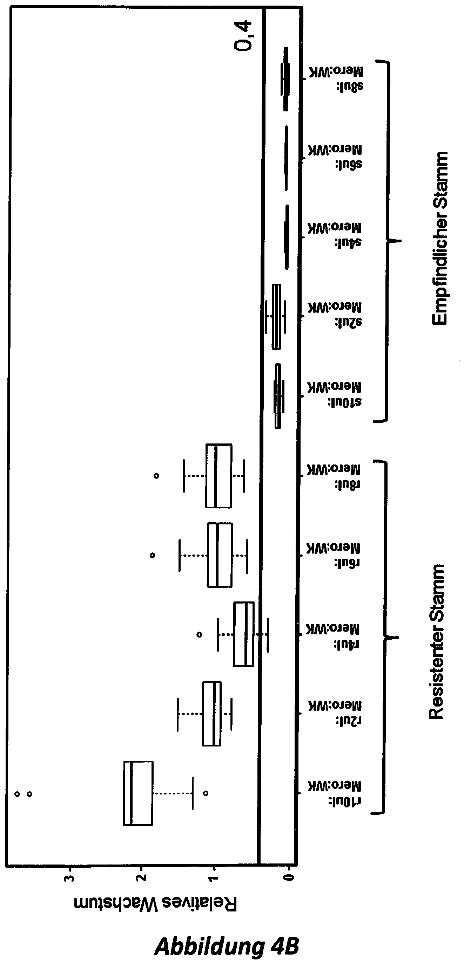

**Abbildung 4B** verdeutlicht in einem Balkendiagramm eine Statistik des Wachstumsverhaltens von *Klebsiella pneumoniae* gegenüber Meropenem aus dem Experiment aus **Abbildung 4A** über fünf verschiedene Tropfengrößen 2, 4, 6, 8 und 10 Mikroliter. Wie zu erkennen ist, liegt das relative Wachstum beim empfindlichen Stamm verlässlich unter dem Schwellwert signifikantem Wachstums von 0,4, wohingegen es beim resistenten Stamm weit über dem Schwellwert landet; bis auf eine Ausnahme bei den vier Mikroliter-Tropfen, wo der Median zwar deutlich größer als 0,4 ist, jedoch auch vereinzelt Messungen darunter auftreten.

Die Resistenz/Empfindlichkeitstestung kann an mikrobiellen Proben, die direkt aus Kulturen oder direkt aus biologischem Material gewonnen wurden, ausgeführt werden. Üblicherweise werden im Stand der Technik für die Empfindlichkeitstestung reife Kulturen eingesetzt, die zum Beispiel 16-24 Stunden auf einem Festmedium wie Agar inkubiert wurden und nach derartigen Bebrütungszeiten in Form von ausgebildeten Kolonien vorliegen. Auch die Testung aus reifen, in Flüssignährmedium bebrüteten Kulturen ist möglich.

Allerdings ist es in vielen Situationen vorteilhaft, bereits direkt aus dem Untersuchungsmaterial eine Empfindlichkeitstestung durchzuführen, um die Zeit bis zum Ergebnis deutlich zu reduzieren. Als Beispiel für ein derartiges Material mit hoher Bedeutung für eine schnelle Erregerdiagnostik können positive Blutkulturen genannt werden. Der Ablauf der Blutkulturdiagnostik ist heutzutage üblicherweise derart, dass die vom Patienten abgenommenen Blutproben zuerst in spezielle Blutkulturflaschen mit Flüssignährmedium eingebracht werden. Diese Flaschen werden anschließend in automatisierte Inkubatoren eingelesen, welche die Flaschen auf ein eventuelles mikrobielles Wachstum kontinuierlich überwachen; beispielsweise per Kohlendioxidmessung. Bei der Positivmeldung einer Blutkulturflasche wird die Flüssigkeit daraus auf Festmedien ausgestrichen und die letzteren anschließend in der Regel 16-24 Stunden inkubiert. Die daraus resultierenden Kolonien werden für die Identifizierung und Antibiotika-Empfindlichkeitstestung eingesetzt. Unter anderem sind die Kolonien auch für die hier beschriebene Methode der Empfindlichkeitstestung geeignet.

Zeitsparend ist allerdings die Identifizierung und Empfindlichkeitstestung direkt aus positiv-gemeldeten Blutkulturen, um die Zeit für die Anzucht auf Festmedien einzusparen und damit das Ergebnis ca. einen Tag früher zu erlangen. Um das zu erreichen, ist eine Vorverarbeitung der Probe für eine Anreicherung der Mikroorganismen notwendig. Das kann zum Beispiel durch ein Lyse/Zentrifugations-Verfahren oder Lyse/Filtrations-Verfahren erreicht werden. Bei dem Lyse/Zentrifugations-Verfahren werden beispielsweise die Blutzellen zuerst durch Zugabe eines Lysemittels wie einem Tensid lysiert und anschließend werden die Mikroorganismen durch Zentrifugation konzentriert. In einem optionalen Wasch-Schritt wird ein Waschpuffer zugegeben und die Mikroorganismen werden erneut durch Zentrifugation konzentriert. Anschließend erfolgt die Identifizierung entweder sofort oder nach einer Protein-Extraktion. Ein solches Verfahren für die Identifizierung wurde als Identifizierungs-Kit MALDI Sepsityper^{®} (Bruker Daltonik GmbH, Bremen, Deutschland) entwickelt und ist kommerziell erhältlich (N. G. Morgenthaler et al., International Journal of Microbiology Volume 2015, Article ID 827416, 10 pages).

Diese oder ähnliche Verfahren können ebenfalls als Vorverarbeitung der Proben (Anreicherung der Mikroorganismen) für die hier beschriebene Resistenz/Empfindlichkeitstestung mittels MALDI-TOF MS verwendet werden. Dies ermöglicht eine deutliche Reduktion der Zeit bis zum Ergebnis.

Als Alternative können sehr kurz auf Festmedium bebrütete Sub-Kulturen aus positiven Blutkulturen oder anderen Materialien für die hier beschriebene MALDI-TOF MS-basierte Empfindlichkeitstestung verwendet werden. Die Verwendung von sehr kurz auf Festmedium bebrüteten Sub-Kulturen aus positiven Blutkulturen wurde vor kurzem für die Identifizierung (Idelevich et al., Clin Microbiol Infect. 2014; 20:1001-1006) und Empfindlichkeitstestung (Idelevich et al., J Clin Microbiol. 2014; 52:4058-4062) demonstriert. Dabei werden die Festmedien nach Sub-Kultivieren (Ausstreichen) nur kurz, in der Regel 1,5 bis 6 Stunden, inkubiert und die entstehende "junge" mikrobielle Biomasse für die Identifizierung und Empfindlichkeitstestung eingesetzt. Dieses Vorgehen ermöglicht zwar keine direkte Testung unmittelbar nach Positivmeldung einer Blutkultur, ist aber trotzdem sehr schnell im Vergleich zu klassischer Testung aus reifen, über 16-24 Stunden bebrüteten Kolonien. Der Vorteil dieser Methode besteht darin, dass keine zusätzlichen Verbrauchsmaterialien oder zusätzlicher Arbeitsaufwand notwendig sind; es erfolgt lediglich eine frühere Beobachtung der Festmedien und die Testung aus der "jungen" Biomasse.

Besonders vorteilhaft ist die Empfindlichkeitstestung direkt aus Blut ohne vorherige Bebrütung der Blutproben in einem Blutkultur-Automaten. Die Vorverarbeitung der Proben für die Anreicherung der Mikroorganismen kann dabei wie oben für die Testung aus positiv-gemeldeten Blutkulturen beschrieben erfolgen.

Während eine direkte MALDI-TOF MS-basierte Identifizierung direkt aus Blut selbst nach Konzentration der Mikroorganismen wegen der geringen Konzentrationen der Mikroorganismen-Zellen im Blut ohne vorherige Anzucht zurzeit schwer durchführbar ist, ist eine direkte MALDI-TOF MS-basierte Empfindlichkeitstestung direkt aus Blut mittels des hier beschriebenen Verfahrens möglich. Nach Isolieren der Mikroorganismen aus dem Blut wird eine Mikroorganismen-Suspension in einem Flüssignährmedium aufbereitet und, wie bei der Empfindlichkeitstestung üblich, mit einem Antibiotikum gemischt. Anschließend wird diese Suspension und ggf. eine Wachstumskontrolle in Form von Tröpfchen auf einen MALDI-TOF MS-Träger aufgetragen und direkt dort bebrütet. Selbst bei sehr niedrigen initialen mikrobiellen Zellzahlen im Blut werden sich die Mikroorganismen zumindest in der Wachstumskontrolle oder bei Vorliegen einer phänotypischen Resistenz auch in der Mischung aus Probe und Antibiotikum nach einer bestimmten Bebrütungszeit vermehren, was durch das MALDI-TOF Massenspektrometer detektiert werden kann. Die Empfindlichkeitstestung erfolgt also an sich nach dem gleichen Prinzip wie sonst in Bezug auf den ersten bevorzugten Aspekt dieser Offenbarung beschrieben.

Eine Identifizierung von Mikroorganismen ist mittels MALDI-TOF aus reifen, auf Festmedien bebrüteten Kolonien ohne weiteres möglich. Allerdings ist für eine direkte Identifizierung aus Untersuchungsmaterial (zum Beispiel aus positiven Blutkulturen) eine Vorverarbeitung der Proben notwendig. Dies ist beispielsweise mit dem oben beschriebenen Lyse/Zentrifugations-Verfahren möglich. Allerdings erfordert dieses Verfahren zusätzliche Verarbeitungsschritte, was Zeit kostet und die Integration in die Laboratoriums-Routinediagnostik erschwert.

Die in dieser Anmeldung beschriebene Methoden der Detektion und Identifizierung aus Tröpfchen bzw. der Empfindlichkeitstestung in Tröpfchen direkt auf einem MALDI-TOF MS-Träger gemäß dem ersten bevorzugten Aspekt können nicht nur isoliert sondern auch in Kombination ausgeführt werden, wobei eine derartige Kombination insbesondere bei der Testung direkt aus dem Untersuchungsmaterial sinnvoll ist; zum Beispiel aus positiven Blutkulturen. Bei einer derartigen Kombination der Empfindlichkeitstestung mit der Identifizierung wird bei der MALDI-TOF MS-Messung nicht nur das Wachstum der Kontrollmessung mit dem Wachstum der mit einem Antibiotikum versetzten Probe entsprechend der beschriebenen Algorithmen für die Empfindlichkeitstestung verglichen, sondern das ungehemmte mikrobielle Wachstum in der Kontrollmessung lässt sich zusätzlich auch für die übliche MALDI-TOF MS-Identifizierung verwenden. Bei ausreichend langen Inkubationszeiten, die aber immer noch im Vergleich zu üblichen Inkubationszeiten von16-24 Stunden sehr kurz sind, ist die Menge der mikrobiellen Biomasse für die Identifizierung ausreichend. Die Vorteile dieser kombinierten Methode sind, dass (i) auf zusätzliche Verarbeitungsschritte für beispielsweise die Lyse/Zentrifugations-Methode verzichtet werden kann, (ii) die Ergebnisse von Empfindlichkeitstestung und Identifizierung zeitnah und gleichzeitig zur Verfügung stehen und (iii) die Zeit bis zur abgeschlossenen Empfindlichkeitstestung und Identifizierung kürzer im Vergleich zur klassischen Testung aus reifen Kolonien ist.

Die Anwendung dieser kombinierten Methode kann die Testung aus reifen oder jungen Kolonien wie auch die Testung direkt aus Material betreffen; zum Beispiel aus positiver Blutkultur oder Blut.

Mit einer weiteren Ausführungsform der hier beschriebenen Methoden wird eine schnelle und einfache Detektion von Resistenzmechanismen von Mikroorganismen ermöglicht. Dies wird zum Beispiel durch die Kombinationstestungen erreicht. D.h. es wird zusätzlich zur Suspension bestehend aus Mikroorganismus und Antibiotikum und einer Suspension bestehend nur aus dem Mikroorganismus (Wachstumskontrolle ohne Antibiotikum) auch eine Suspension bestehend aus Mikroorganismus, Antibiotikum und einer Substanz, die eine etwaige Resistenzwirkung des mikrobiellen Organismus gegen das Antibiotikum spezifisch (also basierend auf einem bestimmten Resistenzmechanismus) aufhebt, getestet.

Ein Beispiel dafür ist die Detektion der Bildung von β-Laktamasen durch Bakterien. β-Laktamasen sind bakterielle Enzyme, die β-Laktam-Antibiotika spalten und dadurch unwirksam machen können. Beispiele für β-Laktamasen sind ampC-β-Laktamasen, Extended Spectrum β-Laktamasen (ESBL), Carbapenemasen u.a. Jede Art der β-Laktamasen spaltet ein spezifisches Spektrum an Antibiotika, und hat außerdem unterschiedliche Eigenschaften (zum Beispiel Verortung des Gens auf einem Plasmid oder auf dem Chromosom), die das Angebot an Antibiotika, das für eine Therapie zur Verfügung steht, unterschiedlich stark einschränken sowie unterschiedliche Ausbreitungsgeschwindigkeiten entsprechender bakterieller Stämme ermöglichen. Daher kann eine schnelle Bestimmung des zu Grunde liegenden Resistenzmechanismus von großer Bedeutung sein, insbesondere auch im Zusammenhang mit krankenhaushygienischen Untersuchungen und ggf. einzuleitenden Hygiene-Maßnahmen.

Durch die Zugabe eines spezifischen β-Laktamase-Hemmers (zum Beispiel Clavulansäure für ESBL oder Vaborbactam für Meropenem) kann die Wirkung einer β-Laktamase spezifisch aufgehoben werden. Dieses Prinzip wird nicht nur therapeutisch, sondern auch diagnostisch für die Detektion der β-Laktamase, die der Resistenz zu Grunde liegt, ausgenutzt. Kommerziell stehen beispielsweise mit Antibiotikum getränkte Testblättchen und mit Antibiotikum plus β-Laktamase-Hemmer getränkte Testblättchen zur Verfügung. Nach dem Ausstreichen der Bakterienkultur auf einem Festmedium wie einer Agarplatte werden solche Testblättchen aufgelegt; und nach 16-24 Stunden werden die Hemmhöfe ausgemessen (Agardiffusionstest). Das Erreichen einer bestimmten Differenz der Hemmhof-Durchmesser zwischen dem Testblättchen mit Antibiotikum und dem Testblättchen mit Antibiotikum plus dem β-Laktamase-Hemmer spricht für die Produktion einer bestimmten β-Laktamase.

Der Vorteil der hier beschriebenen Methoden für die Kombinationstests besteht neben der einfacheren Durchführung vor allem darin, dass das Ergebnis bereits nach wenigen Stunden vorliegt im Vergleich zu dem Ergebnis beispielsweise der Agardiffusionsmethode, die einen Zeitaufwand von deutlich mehr als 12 Stunden erfordert, so dass es erst sehr viel später am nächsten Tag vorliegt. Der Geschwindigkeitsvorteil bei den hier beschriebenen Verfahren ergibt sich daraus, dass zum einen das Wachstum der Mikroorganismen in einem Flüssignährmedium deutlich schneller ist als auf einem Festmedium, und zum anderen in einem Tröpfchen eine hohe Mikroorganismen-Konzentration durch das geringe Flüssigkeitsvolumen schnell erreicht wird. Zum dritten gewährleistet die massenspektrometrische Messung, zum Beispiel per MALDI-TOF MS, eine empfindlichere und schnellere Wachstumsdetektion, als sie durch visuelle Betrachtung des Wachstums auf einem Festmedium wie im Fall der Agardiffusionsmethode erzielbar ist.

Im Vergleich zu dem eingangs erwähnten Nachweis von β-Laktamasen durch die Detektion der β-Laktam-Spaltung mittels MALDI-TOF MS (Massensignale von ungespaltenem β-Laktam bzw. von Spaltprodukten) hat die hier beschriebene MALDI-TOF MS-basierte Methode unter Verwendung der Kombinationstests einen wichtigen Vorteil: Bei Detektion der β-Laktam-Spaltung geht es um eine indirekte Methode, d.h. es wird im positiven Fall gezeigt, dass ein (3-Laktam-Antibiotikum gespalten wird und es wird abgeleitet, dass das Antibiotikum für diesen Bakterienstamm nicht wirksam sein wird. Die Wirksamkeit kann aber noch von anderen Faktoren abhängig sein, wie zum Beispiel von der Dosis des Antibiotikums. Bei dem hier beschriebenen Kombinationstest ermittelt man zusätzlich direkt die Wirkung des β-Laktamase-Hemmers auf das Wachstum des Mikroorganismus, d.h. ob es zu einer Aufhebung der Resistenz kommt oder nicht. Derartige Ergebnisse haben eine deutlich höhere klinische Relevanz.

In Anlehnung an die vorherigen Ausführungen können die hier beschriebenen Kombinationstests für die Testung aus reifen oder jungen Kolonien wie auch für die Testung direkt aus Material angewendet werden; zum Beispiel aus positiver Blutkultur oder Blut.

Außer der Anwendung der beschriebenen Methoden in Form von einzelnen Schnelltests, d.h. der Testung eines bestimmten Antibiotikums gegen einen bestimmten Mikroorganismus, ist es auch möglich, gleichzeitig mehrere Antibiotika zu testen (Multiplex-Testungen). Das hat den Vorteil, dass gleichzeitig ein komplettes Antibiogramm für die Mikroorganismen in der mikrobiellen Probe erstellt werden kann. Außerdem ist es möglich, gleichzeitig mehrere Konzentrationen für jedes Antibiotikum zu testen, was die Ermittlung der minimalen Hemmkonzentration (MHK) ermöglicht. Unter einer MHK versteht man die minimale Konzentration eines Antibiotikums, die das mikrobielle Wachstum hemmt. Die MHK ist ein Maß für die Empfindlichkeit von Mikroorganismen gegenüber Antibiotika. Zum einen ermöglicht die MHK eine Kategorisierung eines Mikroorganismus in die Kategorien "empfindlich", "intermediär" oder "resistent"; zum anderem gibt die MHK eine Aussage über den "Empfindlichkeitsgrad" eines Mikroorganismus gegen ein bestimmtes Antibiotikum. Für die multiplen Testungen können viele Flecken eines MALDI-TOF MS-Trägers parallel belegt werden, wobei zum Beispiel Träger mit 96, 384 oder auch 1536 Flecken zur Anwendung kommen können.

Das Wachstum der Mikroorganismen kann durch unterschiedliche Auswerte-Algorithmen festgestellt werden. Dabei kann das Wachstum der Mikroorganismen mit Antibiotikum mit dem Wachstum der Mikroorganismen ohne Antibiotikum (Wachstumskontrolle) verglichen werden.

Als ein möglicher Algorithmus wird die Detektion der mikrobiellen Biomasse vorgeschlagen. Für das MALDI-TOF MS-Verfahren ist eine bestimmte untere Nachweisgrenze charakteristisch, d.h. die minimale Menge an mikrobieller Biomasse (etwa 10⁴ oder 10⁵ mikrobielle Zellen pro Fleck), die eine Detektion im Sinne einer Erzeugung von erkennbaren Massensignalen im Massenspektrum ermöglicht. Diese untere Nachweisgrenze ist von vielen Faktoren abhängig, unter anderem von den Geräteigenschaften und Einstellungen. Nach dem hier beschriebenen Algorithmus kann ein Mikroorganismus im Flüssignährmedium in einer Konzentration (Menge) auf einen Fleck aufgetragen werden, die geringer als die untere Nachweisgrenze des MALDI-TOF MS Messverfahrens ist. Das heißt, würde man ohne weitere Prozessierung dieser mikrobiellen Probe eine MALDI-TOF MS-Messung durchführen, könnte im Massenspektrum keine Mikroorganismensignatur über dem allgegenwärtigen Hintergrund nachgewiesen werden. Daraus folgt unmittelbar, dass im Falle der Empfindlichkeit des Mikroorganismus gegenüber dem zu testenden Antibiotikum das Wachstum gehemmt wird und die mikrobielle Biomasse auch nach der Bebrütungszeit kaum zu detektieren sein wird, da die untere Nachweisgrenze nicht überschritten wird. Im Fall der Resistenz des Mikroorganismus gegenüber dem zu testenden Antibiotikum hingegen können die Mikroorganismen genau wie bei der Wachstumskontrolle (ohne Antibiotikum) während der Inkubation wachsen, und die mikrobielle Masse lässt sich detektieren, d.h. es kommt zum Nachweis von entsprechenden spezifischen mikrobiellen Massensignalen im Massenspektrum.

Um die Genauigkeit des Verfahrens zu erhöhen und die Wahrscheinlichkeit der Fehlinterpretation einer "zufällig" auftretenden Signatur im Massenspektrum, die einer spezifischen mikrobiellen Massensignal-Signatur ähnelt, selbst bei kleinen Mengen der mikrobiellen Biomasse (normalerweise unter der Nachweisgrenze) zu verhindern, kann (ggf. zusätzlich in Kombination) die Quantifizierung bzw. die relative Quantifizierung der Menge an mikrobieller Biomasse verwendet werden. Das lässt sich beispielsweise durch einen Vergleich der sogenannten "Area Under the Curve" (AUC) und/oder von Peak-Intensitäten durch Verwendung eines internen Standards ("MBT-ASTRA", Bruker Daltonik GmbH, Bremen, Deutschland) oder durch andere statistische Methoden erreichen, die dem Fachmann grundsätzlich vertraut sind und hier nicht weiter erläutert werden müssen. Insbesondere lässt sich der Referenzdatensatz für den Abgleich mit einer mikrobiellen Massensignatur im aufgenommenen Massenspektrum aus den Massensignalen eines internen Standards oder einer Referenzsubstanz im gleichen Massenspektrum ableiten bzw. ermitteln.

Als andere mögliche Variante wird der Algorithmus der räumlichen Auflösung vorgeschlagen. Dabei werden durch MALDI-TOF MS-Verfahren die Laser-Schüsse in einem genau definierten räumlichen Raster abgegeben - zum Beispiel 1.000 Schüsse verteilt auf jeweils definierte Bereiche eines präparierten Flecks eines MALDI-TOF MS-Trägers. Die Anzahl der "erfolgreichen" Schüsse, d.h. Schüsse bei denen ein Massenspektrum mit nachweisbarer Mikroorganismensignatur generiert wurde, wird beispielsweise zwischen der mikrobiellen Probe mit Antibiotikum und der mikrobiellen Probe ohne Antibiotikum (Wachstumskontrolle) verglichen.

Dieser Algorithmus kann zum Beispiel auch als Ergänzung zum oben beschriebenen Algorithmus der Detektion der mikrobiellen Biomasse verwendet werden, um die Genauigkeit des Detektionsverfahrens zu erhöhen und die Wahrscheinlichkeit zu verringern, dass "zufällig" auftretende Signaturen im Massenspektrum selbst bei kleinen Mengen der mikrobiellen Biomasse (normalerweise unter der Nachweisgrenze) als signifikantes Wachstum fehlinterpretiert werden. D.h. eine geringe Anzahl der erfolgreichen Schüsse lässt sich beispielsweise noch nicht als Wachstum interpretieren, sondern wird als zufällig und nicht signifikant gedeutet.

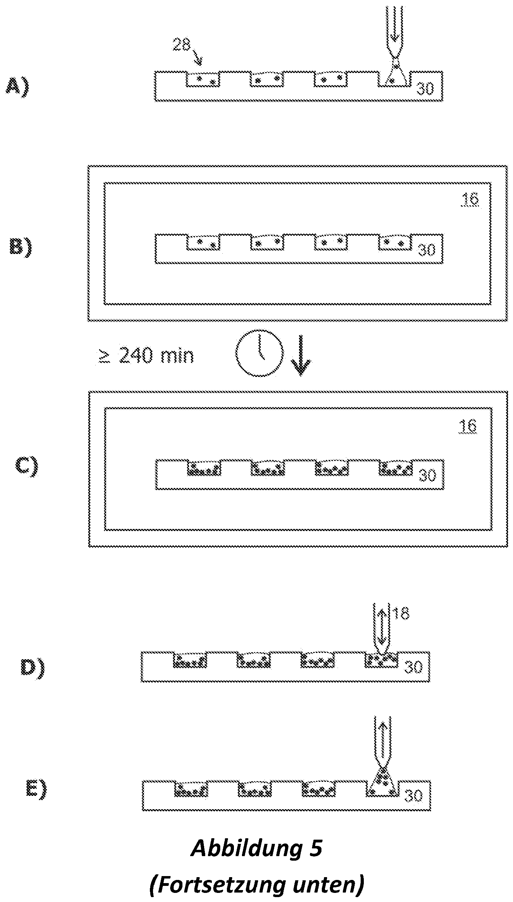

Abbildung 5A bis **Abbildung 5I** erläutern ein Ausführungsbeispiel eines Verfahrens, das nicht dem Schutzbereich der beigefügten Patentansprüche unterfällt und der Veranschaulichung dient. Da viele Schritte denjenigen der zuvor geschilderten Verfahren ähneln, so dass die diesbezüglichen Ausführungen auch auf dieses Beispiel angewendet werden können, beschränkt sich die nachfolgende Beschreibung in aller gebotenen Kürze auf die wesentlichen Unterschiede zu den Verfahren gemäß dem ersten bevorzugten Aspekt der Offenbarung.

Ein wesentlicher Unterschied besteht darin, dass die Züchtung/Bebrütung von Mikroorganismen und die Präparation für eine massenspektrometrische Messung nicht auf dem gleichen flachen Substrat wie einem massenspektrometrischen Probenträger sondern auf getrennten Substraten (oder Substratabschnitten) stattfinden. Eine Mikroorganismen-Suspension in einem flüssigen Nährmedium wird in Gefäße 28 eingegeben; zum Beispiel Näpfe in einer Mikrotiterplatte 30. Das Inokulum kann etwa 10⁶ cfu pro Milliliter betragen; das Volumen des Nährmediums etwa 50 bis 250 Mikroliter; bevorzugt 100 Mikroliter. Die Näpfe 28 können (zum Beispiel als Testkit) für eine Resistenz/Empfindlichkeitstestung bereits vor der Zugabe einer Mikroorganismus-Suspension antimikrobielle Substanzen enthalten, beispielsweise in Form einer Lösung, eines Pulvers, oder in einer lyophilisierten Form. Alternativ lassen sich diese Antibiotika dem Nährmedium auch später beimengen. **Abbildung** 5A.

Die Napfplatte 30 wird in einen Brutschrank 16 verbracht und dort für eine bestimmte Inkubationszeit von beispielsweise 4 bis 18 Stunden aufbewahrt, um Mikroorganismenwachstum anzuregen. Die Mikroorganismen neigen, wie bereits erläutert, zur Bildung von Ablagerungen ("Mikroorganismen-Biofilm") am Grund sowie dem unteren Teil der Seitenwände der Näpfe 28. **Abbildungen 5B-5C.**

Die Napfplatte 30 wird dem Brutschrank 16 entnommen. Um den Näpfen 28 ein Volumen des Nährmediums mit ausreichender Menge an intakten gewachsenen Mikroorganismen aus einer annähernd gleichmäßigen Mikroorganismen-Verteilung entnehmen zu können, kann die Ablagerung beispielsweise durch mehrmaliges Auf- und Abbewegen der Pipettenspitze 18 oder durch sachtes Agitieren der Napfplatte 30 kurz vor der Entnahme aufgewirbelt werden, so dass die Mikroorganismen in größerer Konzentration und gleichmäßig verteilt mit der Flüssigkeit des Nährmediums beprobt werden können. Die entnommene Flüssigkeitsmenge kann zwischen 1 und 10 Mikrolitern betragen. Abbildung 5D-5E.

Alternativ zu dieser Vorgehensweise kann gegebenenfalls die Bildung einer Mikroorganismenablagerung in den Näpfen 28 während der Züchtung von vornherein behindert oder aufgehalten werden, indem die Napfplatte 30 während der Zeit im Brutschrank 16 behutsam agitiert wird (nicht dargestellt). Dann können das Aufwirbeln mittels der Pipettenspitze 18 und/oder ein nachträgliches Agitieren entbehrlich sein.

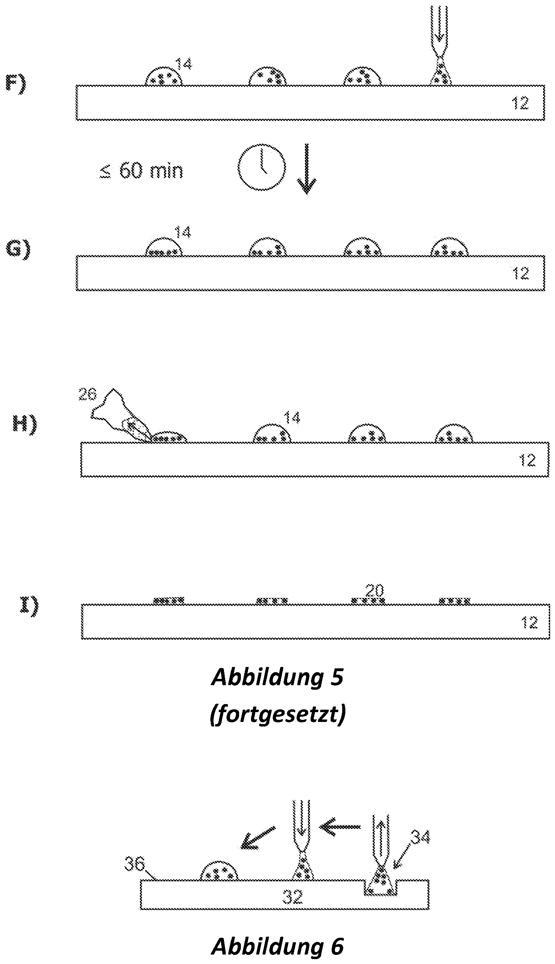

Die entnommene Flüssigkeitsmenge mit den darin enthaltenen intakten Mikroorganismen wird als Tropfen 14 auf den Flecken eines flachen massenspektrometrischen Probenträgers 12 abgelegt. **Abbildung 5F****.**

Es wird eine Steh- oder Ruhezeit von etwa 10 bis 60 Minuten abgewartet, in welcher den Mikroorganismen Gelegenheit gegeben wird, sich an der Schnittstelle zwischen Tropfenflüssigkeit und Trägeroberfläche anzulagern bzw. dort zu sedimentieren. Grundsätzlich gilt, dass mit zunehmender Mikroorganismenkonzentration in dem Tropfen 14 auf dem Probenträger 12 die Stehzeit verringert werden kann; mit anderen Worten: bei hoher Konzentration kann die Stehzeit am unteren Ende des bevorzugten Intervalls liegen; bei niedriger Konzentration kann es vorteilhaft sein, eine längere Zeit abzuwarten. **Abbildung 5G****.**

Wie zuvor schon in anderem Zusammenhang erläutert, kann nach der Stehzeit Restflüssigkeit des Nährmediums vom Probenfleck entfernt werden, beispielsweise mittels eines saugfähigen Gewebes (Tuch 26), das seitlich an der Trägeroberfläche mit dem Tropfen 14 auf einem Fleck in Fluidkontakt gebracht wird und einen Großteil der Flüssigkeit einfach absaugt. Es sind aber natürlich auch andere Arten der Flüssigkeitsentfemung wie das Abpipettieren anwendbar, wie zuvor bereits geschildert. **Abbildung 5H****.**

Die auf diese Weise freigelegte Mikroorganismenablagerung 20 kann jetzt wie zuvor beschrieben weiter präpariert und in einem Massenspektrometer vermessen werden. Zum Beispiel können Peptide/Proteine der Mikroorganismen extrahiert und/oder die Ablagerung 20 gewaschen und/oder die Ablagerung 20 in eine MALDI-Matrixsubstanz eingebettet werden. **Abbildung 5I****.**

**Abbildung 6** veranschaulicht schematisch und beispielhaft eine kombinierte Napf/Probenträgerplatte 32 mit einer Vertiefung 34 (die stellvertretend für eine Vielzahl von Vertiefungen stehen kann), in der die Mikroorganismen gezüchtet werden können, und einem davon beabstandeten flachen Abschnitt 36 mit Probenflecken, der als Substrat für eine massenspektrometrische Probenpräparation verwendet werden kann. In der Ionenquelle des Massenspektrometers kann die durch die Vertiefung 34 hervorgerufene Störung des elektrischen Feldes beispielsweise durch vorheriges bündiges Abdecken der Vertiefung 34 gemindert werden (nicht dargestellt).

Die hier beschriebenen Prinzipien sind nicht zwingend auf MALDI-TOF MS-Messverfahren beschränkt, sondern können grundsätzlich auch mit anderen Nachweis- bzw. Differenzierungsmethoden umgesetzt werden, wie zum Beispiel mit anderen massenspektrometrische Nachweismethoden oder Methoden zur Bestimmung der intrinsischen Fluoreszenz.

Neben den beispielhaft erläuterten Ausführungen sind noch weitere Ausführungsformen der Erfindung denkbar. In Kenntnis dieser Offenbarung ist es dem Fachmann ohne weiteres möglich, weitere vorteilhafte Aufbereitungs- und massenspektrometrische Messverfahren für lebendige, mikrobielle Proben und Mikroorganismen zu entwerfen.

## Patentansprüche

1. Verfahren zur Aufbereitung lebendiger, mikrobieller Proben für eine anschließende massenspektrometrische Messung, aufweisend die Schritte:
(a) Bereitstellen eines flachen Probenträgers, der mehrere Probenflecken umfasst;
(b) Aufbringen wenigstens einer lebendigen, mikrobiellen Probe in einem Nährmedium-Tropfen auf wenigstens einen der Probenflecken;
(c) Verbringen des Probenträgers in einen Bebrütungsraum mit definierter Atmosphäre für eine vorbestimmte Zeitspanne, um Mikroorganismenwachstum anzuregen;
(d) Entfernen von Restflüssigkeit des Nährmedium-Tropfens nach der vorbestimmten Zeitspanne, um eine Mikroorganismenablagerung auf dem Probenfleck freizulegen;
(e) Präparieren des Probenflecks für eine desorbierende Ionisierung;
(f) Überführen des Probenträgers in eine Desorptions-Ionenquelle eines Massenspektrometers, Erzeugen von Ionen aus dem präparierten Probenfleck und Aufnehmen wenigstens eines zugehörigen Massenspektrums; und
(g) Abgleichen des aufgenommenen Massenspektrums mit einem Referenzdatensatz, um wenigstens eine Eigenschaft der mikrobiellen Probe zu ermitteln.

2. Verfahren nach Anspruch 1, bei dem der Referenzdatensatz Referenzspektren aufweist, die einer Bibliothek früher aufgenommener Massenspektren entnommen werden, wobei die wenigstens eine Eigenschaft aus Schritt (g) im Rahmen einer Identifizierung Art oder Unterart von Mikroorganismen in der mikrobiellen Probe umfasst.

3. Verfahren nach Anspruch 1 oder 2, bei dem die gleiche mikrobielle Probe in Schritt (b) parallel auf mehrere Probenflecken aufgebracht wird und die Nährmedium-Tropfen mal eine antimikrobielle Substanz oder eine Kombination aus antimikrobieller Substanz und Enzym-hemmer enthalten und mal nicht.

4. Verfahren nach Anspruch 3, bei dem ein β-Laktamase-Hemmer als Enzym-Hemmer verwendet wird.

5. Verfahren nach Anspruch 3 oder Anspruch 4, bei dem der Referenzdatensatz ein zeitnah aufgenommenes Massenspektrum eines Probenflecks ist, auf den in Schritt (b) ein Nährmedium-Tropfen ohne antimikrobielle Substanz oder Kombination aus antimikrobieller Substanz und Enzym-Hemmer aufgebracht worden ist, und die wenigstens eine Eigenschaft aus Schritt (g) im Rahmen einer Charakterisierung eine Empfindlichkeit von Mikroorganismen in der mikrobiellen Probe gegenüber der antimikrobiellen Substanz bzw. der Kombination aus antimikrobieller Substanz und Enzym-Hemmer umfasst.

6. Verfahren nach Anspruch 5, bei dem in Schritt (b) mehrere Nährmedium-Tropfen mit jeweils verschiedenen Konzentrationen der gleichen antimikrobiellen Substanz aufgebracht werden und die wenigstens eine Eigenschaft aus Schritt (g) im Rahmen einer Charakterisierung eine minimale Hemmkonzentration der antimikrobiellen Substanz bezüglich der Mikroorganismen umfasst.

7. Verfahren nach einem der Ansprüche 3 bis 6, bei dem die wenigstens eine Eigenschaft in Schritt (g) aus einem Unterschied im Mikroorganismenwachstum abgeleitet wird.

8. Verfahren nach einem der Ansprüche 3 bis 7, bei dem die mikrobielle Probe in Schritt (b) in dem Nährmedium-Tropfen derart bemessen wird, dass eine Menge leicht unterhalb der Nachweisgrenze der massenspektrometrischen Messung liegt.

9. Verfahren nach einem der Ansprüche 1 bis 8, bei dem Temperatur und Feuchtigkeit in dem Bebrütungsraum in Schritt (c) auf etwa 36°C bzw. nahe der Sättigung eingestellt werden.

10. Verfahren nach einem der Ansprüche 1 bis 9, bei dem das Entfernen von Restflüssigkeit in Schritt (d) ein Abtupfen eines Tropfenüberstandes mittels eines saugfähigen Materials oder ein Abpipettieren umfasst.

11. Verfahren nach einem der Ansprüche 1 bis 10, bei dem das Präparieren in Schritt (e) eine vorbereitende Extraktion mikrobieller Proteine/Peptide aus der Mikroorganismenablagerung und/oder ein Waschen der Mikroorganismenablagerung und/oder eine Einbettung der Mikroorganismenablagerung in eine Laserlicht absorbierende Matrixsubstanz, um anschließend in Schritt (f) per matrix-unterstützter Laserdesorption (MALDI) ionisiert zu werden, umfasst.

12. Verfahren nach einem der Ansprüche 1 bis 11, bei dem die Massenspektren in Schritt (f) Flugzeit-dispersiv aufgenommen werden.

13. Verfahren nach einem der Ansprüche 1 bis 12, bei dem in Schritt (b) die mikrobielle Probe (i) als Suspension in dem Nährmedium-Tropfen auf dem wenigstens einen Probenflecken dispensiert wird oder (ii) zuerst in Zellform auf dem wenigstens einen Probenflecken abgelegt und anschließend in einen dispensierten Nährmedium-Tropfen eingetaucht wird.

## Claims

1. Method for the preparation of living, microbial samples for a subsequent mass-spectrometric measurement, comprising the steps:
(a) providing a flat sample support containing several sample spots;
(b) depositing at least one living, microbial sample in a droplet of nutrient medium on at least one of the sample spots;
(c) placing the sample support into an incubation space with defined atmosphere for a predetermined period of time to stimulate the growth of microorganisms;
(d) removing residual liquid of the droplet of nutrient medium after the predetermined period of time to expose a deposit of microorganisms on the sample spot;
(e) preparing the sample spot for a desorbing ionization;
(f) transferring the sample support into a desorption ion source of a mass spectrometer, generating ions from the prepared sample spot and acquiring at least one corresponding mass spectrum; and
(g) comparing the mass spectrum acquired with a reference data set to determine at least one characteristic of the microbial sample.

2. The method according to Claim 1, wherein the reference data set has reference spectra which are taken from a library of previously acquired mass spectra, and for an identification, the at least one characteristic from Step (g) comprises species or subspecies of microorganisms in the microbial sample.

3. The method according to Claim 1 or 2, wherein the same microbial sample is applied in Step (b) in parallel onto several sample spots, and the droplets of nutrient medium sometimes contain an antimicrobial substance or a combination of antimicrobial substance and enzyme inhibitor and sometimes do not.

4. The method according to Claim 3, wherein a β-lactamase inhibitor is used as the enzyme inhibitor.

5. The method according to Claim 3 or Claim 4, wherein the reference data set is a mass spectrum acquired very recently from a sample spot onto which in Step (b) a droplet of nutrient medium without antimicrobial substance or combination of antimicrobial substance and enzyme inhibitor has been applied; and the at least one characteristic from Step (g) for a characterization comprises a sensitivity of microorganisms in the microbial sample to the antimicrobial substance or the combination of antimicrobial substance and enzyme inhibitor.

6. The method according to Claim 5, wherein in Step (b) several droplets of nutrient medium, each with different concentrations of the same antimicrobial substance, are applied and, for a characterization, the at least one characteristic from Step (g) comprises a minimum inhibitory concentration of the antimicrobial substance with respect to the microorganisms.

7. The method according to any one of Claims 3 to 6, wherein the at least one characteristic in Step (g) is derived from a difference in the microorganism growth.

8. The method according to any one of Claims 3 to 7, wherein the microbial sample in Step (b) is dosed in the droplet of nutrient medium such that a quantity is slightly below the detection limit of the mass-spectrometric measurement.

9. The method according to any one of Claims 1 to 8, wherein temperature and humidity in the incubation space in Step (c) are set to around 36°C and close to saturation, respectively.

10. The method according to any one of Claims 1 to 9, wherein the removal of residual liquid in Step (d) comprises dabbing off the droplet supernatant with an absorbent material or pipetting it off.

11. The method according to any one of Claims 1 to 10, wherein the preparation in Step (e) comprises a preparatory extraction of microbial proteins/peptides from the microorganism deposit and/or washing of the microorganism deposit and/or embedding the microorganism deposit in a laser light-absorbing matrix substance to be subsequently ionized by matrix-assisted laser desorption (MALDI) in Step (f).

12. The method according to any one of Claims 1 to 11, wherein the mass spectra in Step (f) are acquired with time-of-flight dispersion.

13. The method according to any one of Claims 1 to 12, wherein in Step (b) the microbial sample (i) is dispensed as a suspension in the droplet of nutrient medium on the at least one sample spot or (ii) first deposited in cellular form on the at least one sample spot and subsequently immersed into a dispensed droplet of nutrient medium.

## Revendications

1. Méthode de préparation d'échantillons microbiens vivants en vue d'une mesure subséquente par spectrométrie de masse, comprenant les étapes suivantes :
(a) fournir un support d'échantillon plat contenant plusieurs points d'échantillonnage ;
(b) déposer au moins un échantillon microbien vivant dans une gouttelette de milieu nutritif sur au moins une des points d'échantillonnage ;
(c) placer le support d'échantillon dans un espace d'incubation avec une atmosphère définie pendant une période de temps prédéterminée pour stimuler la croissance des micro-organismes ;
(d) éliminer le liquide résiduel de la gouttelette de milieu nutritif après la période prédéterminée pour exposer un dépôt de micro-organismes sur le point d'échantillonnage ;
(e) préparer le point d'échantillonnage pour une ionisation de désorption ;
(f) transférer le support d'échantillon dans une source d'ions de désorption d'un spectromètre de masse, générer des ions à partir de le point d'échantillonnage préparé et acquérir au moins un spectre de masse correspondant ; et
(g) comparer le spectre de masse acquis avec un ensemble de données de référence pour déterminer au moins une caractéristique de l'échantillon microbien.

2. Méthode selon la revendication 1, dans laquelle l'ensemble de données de référence comporte des spectres de référence provenant d'une bibliothèque de spectres de masse acquis précédemment, et pour une identification, l'au moins une caractéristique de l'étape (g) comprend les espèces ou sous-espèces de micro-organismes dans l'échantillon microbien.

3. Méthode selon la revendication 1 ou la revendication 2, dans laquelle le même échantillon microbien est appliqué à l'étape (b) en parallèle sur plusieurs points d'échantillonnage, et les gouttelettes de milieu nutritif contiennent parfois une substance antimicrobienne ou une combinaison de substance antimicrobienne et d'inhibiteur enzymatique et parfois non.

4. Méthode selon la revendication 3, dans laquelle un inhibiteur de β-lactamase est utilisé comme inhibiteur enzymatique.

5. Méthode selon la revendication 3 ou la revendication 4, dans laquelle l'ensemble de données de référence est un spectre de masse acquis très récemment à partir d'un point d'échantillonnage sur lequel, à l'étape (b), une gouttelette de milieu nutritif sans substance antimicrobienne ou combinaison de substance antimicrobienne et d'inhibiteur enzymatique a été appliquée ; et l'au moins une caractéristique de l'étape (g) pour une caractérisation comprend une sensibilité des micro-organismes dans l'échantillon microbien à la substance antimicrobienne ou à la combinaison de substance antimicrobienne et d'inhibiteur enzymatique.

6. Méthode selon la revendication 5, dans laquelle, à l'étape (b), plusieurs gouttelettes de milieu nutritif, contenant chacune différentes concentrations de la même substance antimicrobienne, sont appliquées et, pour une caractérisation, l'au moins une caractéristique de l'étape (g) comprend une concentration minimale inhibitrice de la substance antimicrobienne à l'égard des micro-organismes.

7. Méthode selon l'une quelconque des revendications 3 à 6, dans laquelle l'au moins une caractéristique de l'étape (g) est dérivée d'une différence dans la croissance des micro-organismes.

8. Méthode selon l'une quelconque des revendications 3 à 7, dans laquelle l'échantillon microbien de l'étape (b) est dosé dans la gouttelette de milieu nutritif de manière à ce que la quantité soit légèrement inférieure à la limite de détection de la mesure spectrométrique de masse.

9. Méthode selon l'une quelconque des revendications 1 à 8, dans laquelle la température et l'humidité dans l'espace d'incubation à l'étape (c) sont réglées à environ 36°C et proches de la saturation, respectivement.

10. Méthode selon l'une quelconque des revendications 1 à 9, dans laquelle l'élimination du liquide résiduel à l'étape (d) comprend à tamponner le surnageant des gouttelettes avec un matériau absorbant ou à l'enlever à la pipette.

11. Méthode selon l'une quelconque des revendications 1 à 10, dans laquelle la préparation à l'étape (e) comprend une extraction préparatoire des protéines/peptides microbiens du dépôt de micro-organismes et/ou le lavage du dépôt de micro-organismes et/ou l'incorporation du dépôt de micro-organismes dans une substance matricielle absorbant la lumière laser pour être ensuite ionisée par désorption laser assistée par matrice (MALDI) à l'étape (f).

12. Méthode selon l'une quelconque des revendications 1 à 11, dans laquelle les spectres de masse à l'étape (f) sont acquis avec une dispersion à temps de vol.

13. Méthode selon l'une quelconque des revendications 1 à 12, dans laquelle, à l'étape (b), l'échantillon microbien (i) est distribué sous forme de suspension dans la gouttelette de milieu nutritif sur l'au moins un point d'échantillonnage (ii) est d'abord déposé sous forme cellulaire sur l'au moins un point d'échantillonnage et ensuite immergé dans une gouttelette distribuée de milieu nutritif.
